# EUROPEAN PATENT SPECIFICATION

(11) **EP 3 903 878 B1**
(45) Date of publication and mention of the grant of the patent: **31.01.2024**
(21) Application number: 18944840.0
(22) Date of filing: 27.12.2018
(51) Int. Cl.: A61N 1/39, A61B 5/287, A61N 1/05

(54) **INTRACARDIAC DEFIBRILLATION CATHETER SYSTEM**
KATHETERSYSTEM FÜR INTRAKARDIALE DEFIBRILLATION
SYSTÈME DE CATHÉTER DE DÉFIBRILLATION INTRACARDIAQUE

(43) Date of publication of application: 03.11.2021
(73) Proprietor: Japan Lifeline Co., Ltd., Tokyo 140-0002 (JP)
(72) Inventor: KOJIMA, Yasuhiro, Tokyo 140-0002 (JP)
(74) Representative: Grünecker Patent- und Rechtsanwälte PartG mbB
(86) International application number: PCT/JP2018/048116
(87) International publication number: WO 2020/136798

(56) References cited:
- WO-A1-2018/083842
- WO-A1-2018/083842
- JP-A- 2014 217 636
- JP-B2- 5 900 974
- US-A1- 2007 100 379
- US-B1- 8 600 490
- US-B1- 8 600 490

## Description

### Technical Field

The present invention, as defined in the appended claims, generally relates to an intracardiac defibrillation catheter system, and more specifically to a catheter system including a defibrillation catheter to be inserted into a cardiac cavity, a power supply device that applies a direct-current voltage to electrodes of the defibrillation catheter, and an electrocardiograph.

### Background Art

In the related art, as an intracardiac defibrillation catheter system capable of reliably supplying electrical energy necessary and sufficient for defibrillation to a heart affected by atrial fibrillation or the like during cardiac catheterization and capable of performing defibrillation treatment without causing burns to the body surface of a patient, a catheter system has been proposed by this applicant, which includes a defibrillation catheter to be inserted into a cardiac cavity to perform defibrillation, a power supply device that applies a direct-current voltage to electrodes of the defibrillation catheter, and an electrocardiograph, in which the defibrillation catheter is configured to include an insulating tube member, a first DC electrode group composed of a plurality of ring-shaped electrodes mounted in a distal end region of the tube member, a second DC electrode group composed of a plurality of ring-shaped electrodes mounted on the tube member at positions spaced away toward a proximal end side from the first DC electrode group, a first lead wire group composed of a plurality of lead wires whose distal ends are connected to the respective electrodes constituting the first DC electrode group, and a second lead wire group composed of a plurality of lead wires whose distal ends are connected to the respective electrodes constituting the second DC electrode group; the power supply device is configured to include a DC power supply unit, a catheter connection connector to be connected to a proximal end side of the first lead wire group and the second lead wire group of the defibrillation catheter, an electrocardiograph connection connector to be connected to an input terminal of the electrocardiograph, an arithmetic processing unit that controls the DC power supply unit on the basis of an input of an external switch and that has an output circuit for a direct-current voltage from the DC power supply unit, and a switching unit composed of a one-circuit two-contact switching switch and having a common contact to which the catheter connection connector is to be connected, a first contact to which the electrocardiograph connection connector is to be connected, and a second contact to which the arithmetic processing unit is to be connected; and when a cardiac potential is to be measured with the electrodes of the defibrillation catheter (the electrodes constituting the first DC electrode group and/or the second DC electrode group), the first contact is selected in the switching unit, and cardiac potential information from the defibrillation catheter is input to the electrocardiograph through the catheter connection connector, the switching unit, and the electrocardiograph connection connector of the power supply device, and when defibrillation is to be performed with the defibrillation catheter, the contact of the switching unit is switched to the second contact by the arithmetic processing unit of the power supply device, and voltages having different polarities are applied to the first DC electrode group and the second DC electrode group of the defibrillation catheter from the DC power supply unit through the output circuit of the arithmetic processing unit, the switching unit, and the catheter connection connector (see PTL 1 below).

In the catheter system described in PTL 1, when an energy applying switch, which is the external switch, is input, the contact of the switching unit is switched from the first contact to the second contact by the arithmetic processing unit, and a path from the catheter connection connector to the arithmetic processing unit through the switching unit is ensured.

After the contact of the switching unit is switched to the second contact, direct-current voltages having different polarities are applied to the first DC electrode group and the second DC electrode group of the defibrillation catheter from the DC power supply unit, which has received a control signal from the arithmetic processing unit, through the output circuit of the arithmetic processing unit, the switching unit, and the catheter connection connector.

Here, the arithmetic processing unit performs arithmetic processing so that a voltage is applied in synchronization with a cardiac potential waveform input through an electrocardiogram input connector, and sends a control signal to the DC power supply unit.

To perform effective defibrillation treatment and provide no adverse effect on the ventricles, defibrillation (application of a voltage) is typically performed in synchronization with an R wave.

If defibrillation is performed in synchronization with a T wave, the risk of inducing serious ventricular fibrillation is high, and therefore it is necessary to avoid synchronization with a T wave.

In the catheter system described in PTL 1, accordingly, one R wave is detected in a cardiac potential waveform (electrocardiogram) sequentially input to the arithmetic processing unit to determine its wave height, a peak that reaches 80% of this wave height immediately after an input of the energy applying switch is recognized as an R wave, and voltages are applied to the first electrode group and the second electrode group in synchronization with this peak.

Furthermore, a catheter system has been proposed by this applicant, in which an arithmetic processing unit sequentially senses an event estimated to be an R wave from an electrocardiogram input from an electrocardiograph to a power supply device through an electrocardiogram input connector, and, when the polarity of an event sensed after an input of an electrical energy applying switch matches the polarity of the immediately preceding sensed event and the polarity of the further preceding sensed event, performs arithmetic processing so that voltages are applied to the first electrode group and the second electrode group in synchronization with the event to control the DC power supply unit, and controls the DC power supply unit so that no voltage is applied to the first DC electrode group or the second DC electrode group for a certain period (a period of at least 50 ms and at most 500 ms) after an event estimated to be an R wave is sensed (see PTL 2 below).

According to the intracardiac defibrillation catheter system having such a configuration, no voltage is applied to the first DC electrode group or the second DC electrode group for a certain period after an event estimated to be an R wave is sensed, which is set as a refractory period. Thus, if the sensed event is a peak of an R wave, it is possible to avoid performing defibrillation at the time point when the subsequent T wave appears, or, in other words, it is possible to mask a peak estimated to be a T wave.

Further relevant prior art is described in WO2018/083842 A1, JP5900974 B2, US8600490 B1 and US2007/100379 A1.

### Citation List

### Patent Literature

PTL 1: Japanese Patent No. 4545216
PTL 2: Japanese Patent No. 5900974

### Summary of Invention

### Technical Problem

However, even the catheter system described in PTL 2 described above cannot reliably prevent defibrillation from being performed in synchronization with a T wave.

That is, for a patient having a low heart rate (long R-R time period), a T wave is considered to appear after a lapse of a period specified in PTL 2 after an event estimated to be an R wave is sensed. In such a case, defibrillation is performed in synchronization with the T wave.

Furthermore, for a patient having a high heart rate (short R-R time period), during the period specified in PTL 2 after an event estimated to be an R wave is sensed, the next event (R wave) for which defibrillation is to be performed in synchronization with the sensed event appears. Thus, no defibrillation can be performed in synchronization with an event for which defibrillation is to be performed.

In addition, it is also considered that the heart rate of the patient changes during surgery.

The present invention has been made on the basis of the above circumstances.

It is an object of the present invention to provide an intracardiac defibrillation catheter system capable of reliably avoiding performing defibrillation in synchronization with a T wave without being affected by the level of the heart rate of a patient, and capable of applying a direct-current voltage to electrodes of a defibrillation catheter to perform defibrillation in synchronization with an R wave of an electrocardiogram input to an arithmetic processing unit.

### Solution to Problem

In order to solve the aforementioned problem, it is provided an intracardiac defibrillation catheter system having the features defined in claim 1. Further preferred embodiments are defined in the dependent claims.
(1) An intracardiac defibrillation catheter system of the present invention is a catheter system including a defibrillation catheter to be inserted into a cardiac cavity to perform defibrillation, a power supply device that applies a direct-current voltage to electrodes of the defibrillation catheter, and an electrocardiograph,
   wherein the defibrillation catheter is configured to include an insulating tube member,
   a first electrode group composed of a plurality of ring-shaped electrodes mounted in a distal end region of the tube member,
   a second electrode group composed of a plurality of ring-shaped electrodes mounted on the tube member at positions spaced away toward a proximal end side from the first electrode group,
   a first lead wire group composed of a plurality of lead wires whose distal ends are connected to the respective electrodes constituting the first electrode group, and
   a second lead wire group composed of a plurality of lead wires whose distal ends are connected to the respective electrodes constituting the second electrode group;
   wherein the power supply device is configured to include a DC power supply unit,
   a catheter connection connector to be connected to a proximal end side of the first lead wire group and the second lead wire group of the defibrillation catheter,
   an external switch including an application execution switch for electrical energy,
   an arithmetic processing unit that controls the DC power supply unit on the basis of an input of the external switch, and
   an electrocardiogram input connector to be connected to the arithmetic processing unit and an output terminal of the electrocardiograph;
   wherein defibrillation is performed with the defibrillation catheter in response to the application execution switch being input, and when defibrillation is to be performed, voltages having different polarities are applied to the first electrode group and the second electrode group of the defibrillation catheter from the DC power supply unit through the catheter connection connector; and
   wherein the arithmetic processing unit of the power supply device sequentially senses an event estimated to be an R wave from an electrocardiogram input from the electrocardiograph through the electrocardiogram input connector, calculates and updates a heart rate each time sensing is performed, and, when, after an n-th (n is an integer greater than or equal to 1) event (Vₙ) estimated to be an R wave (except for an event sensed during a refractory period described below caused by a preceding event) is sensed and after the application execution switch is input, an (n+m)-th (m is an integer greater than or equal to 1) event (Vₙ₊ₘ) estimated to be an R wave is sensed, performs arithmetic processing so that voltages are applied to the first electrode group and the second electrode group in synchronization with the event (Vₙ₊ₘ) only in a case where the event (Vₙ₊ₘ) is sensed after a lapse of a refractory period [a refractory period caused by the event (Vₙ)] that begins at a sensing time point of the event (Vₙ) and whose length changes in proportion to a reciprocal of a heart rate (Aₙ) at the sensing time point of the event (Vₙ), to control the DC power supply unit.

According to the intracardiac defibrillation catheter system having such a configuration, after the event (Vₙ) estimated to be an R wave is sensed, even if the event (Vₙ₊ₘ) estimated to be an R wave is sensed during a refractory period caused by the event (Vₙ), no voltage is applied to the first DC electrode group or the second DC electrode group in synchronization with the event (Vₙ₊ₘ). Thus, if the sensed event (Vₙ) is a peak of an R wave, it is possible to avoid performing defibrillation at the time point when the subsequent T wave appears, or, in other words, it is possible to mask a peak estimated to be a T wave.

Additionally, the length of the refractory period caused by the event (Vₙ) changes (increases or decreases) in proportion to the reciprocal of the heart rate (Aₙ) at the sensing time point of the event (Vₙ), and the refractory period is long when the heart rate (Aₙ) is low, whereas the refractory period is short when the heart rate (Aₙ) is high.

Accordingly, even when the heart rate (Aₙ) is low (the R-R time period is long), no T wave appears after a lapse of the refractory period caused by the event (Vₙ), and a T wave can be reliably masked. It is thus possible to reliably avoid performing defibrillation in synchronization with a T wave.

Furthermore, even when the heart rate (Aₙ) is high (the R-R time period is short), during a refractory period caused by the event (Vₙ), the next event for which defibrillation is to be performed in synchronization with this does not appear. Thus, defibrillation can be reliably performed in synchronization with an event (R wave) for which defibrillation is to be performed.

In addition, since the refractory period changes (increases or decreases) on the basis of the heart rate (Aₙ) at the sensing time point of the event (Vₙ), if the heart rate of the patient rapidly changes during surgery, it is possible to cope with such a rapid change.

(2) In the intracardiac defibrillation catheter system of the present invention, the arithmetic processing unit of the power supply device preferably calculates the heart rate (Aₙ) at the sensing time point of the event (Vₙ) on the basis of a plurality of consecutive events including the event (Vₙ) [events (Vₙ), (Vₙ₋₁), (Vₙ₋₂), ..., and (Vₙ₋ᵣ)].

Here, the number of events (r+1) to be sampled for calculating the heart rate (Aₙ) is preferably 2 to 60, and particularly preferably 12.

(3) In the intracardiac defibrillation catheter system of the present invention, the length of the refractory period caused by the event (Vₙ) is preferably a length corresponding to at least 30% and at most 80% of the reciprocal of the heart rate (Aₙ) at the sensing time point of the event (Vₙ).

According to the intracardiac defibrillation catheter system having such a configuration, the refractory period caused by the event (Vₙ) is set to be at least 30% of the reciprocal of the heart rate (Aₙ), whereby even when the heart rate (Aₙ) is low, no T wave appears after a lapse of the refractory period caused by the event (Vₙ), and a T wave can be reliably masked. It is thus possible to reliably avoid performing defibrillation in synchronization with a T wave.

Furthermore, the refractory period caused by the event (Vₙ) is set to be at most 80% of the reciprocal of the heart rate (Aₙ), whereby even when the heart rate (Aₙ) is high, the next event estimated to be an R wave does not appear during the refractory period caused by the event (Vₙ). Thus, it is possible to reliably perform defibrillation in synchronization with an event for which defibrillation is to be performed.

(4) In the intracardiac defibrillation catheter system of the present invention, the length of the refractory period caused by the event (Vₙ) is preferably a length corresponding to 50% of the reciprocal of the heart rate (Aₙ) at the sensing time point of the event (Vₙ).

(5) In the intracardiac defibrillation catheter system of the present invention, preferably, the arithmetic processing unit of the power supply device does not newly sense an event estimated to be an R wave for, as a blanking period, a period of at least 10 ms and at most 500 ms, and preferably a period of 180 ms, after an event estimated to be an R wave is sensed.

According to the intracardiac defibrillation catheter system having such a configuration, since no new event is sensed for a period of at least 10 ms after an event estimated to be an R wave is sensed, if the sensed event is a peak of an R wave and the peak of an S wave that appears subsequently to this peak in an opposite direction increases and reaches a trigger level, it is possible to prevent the continuity of the polarity of the event from being impaired by sensing the peak of this S wave.

Furthermore, even in a case where the sensed event is a peak of an R wave and a T wave appears during a blanking period after the sensing of this event, this T wave is not sensed, whereby it is possible to reliably avoid performing defibrillation in synchronization with this T wave.

(6) In the intracardiac defibrillation catheter system of the present invention, the arithmetic processing unit of the power supply device preferably controls the DC power supply unit so that no voltage is applied to the first electrode group or the second electrode group for a period of at least 10 ms and at most 500 ms, and preferably a period of 260 ms, after an input of the application execution switch.

According to the intracardiac defibrillation catheter system having such a configuration, since no voltage is applied to the first DC electrode group or the second DC electrode group for a period of at least 10 ms after the application execution switch for electrical energy is input, it is possible to prevent noise generated by an input of the application execution switch from being erroneously sensed as an R wave and to prevent defibrillation from being performed in synchronization with this noise.

Furthermore, it is possible to prevent baseline fluctuations caused immediately after an input of the application execution switch from being erroneously sensed as an R wave and to prevent defibrillation from being performed in synchronization with such fluctuations.

(7) Preferably, the intracardiac defibrillation catheter system of the present invention includes cardiac potential measurement means,
the power supply device is configured to include a second electrocardiogram input connector to be connected to the arithmetic processing unit and the cardiac potential measurement means; and
when an electrocardiogram is input from the cardiac potential measurement means through the second electrocardiogram input connector, the arithmetic processing unit of the power supply device sequentially senses an event estimated to be an R wave from the input electrocardiogram, calculates and updates a heart rate each time sensing is performed, and, when, after an n-th event (Vₙ) estimated to be an R wave (except for an event sensed during a refractory period described below caused by a preceding event) is sensed and after the application execution switch is input, an (n+m)-th event (Vₙ₊ₘ) estimated to be an R wave is sensed, performs arithmetic processing so that voltages are applied to the first electrode group and the second electrode group in synchronization with the event (Vₙ₊ₘ) only in a case where the event (Vₙ₊ₘ) is sensed after a lapse of a refractory period [refractory period caused by the event (Vₙ)] that begins at a sensing time point of the event (Vₙ) and whose length changes in proportion to a reciprocal of a heart rate (Aₙ) at the sensing time point of the event (Vₙ), to control the DC power supply unit.

According to the intracardiac defibrillation catheter system having such a configuration, an electrocardiogram can be input from the cardiac potential measurement means to the arithmetic processing unit without passing through the electrocardiograph, and the arithmetic processing unit of the power supply device can also control the DC power supply unit by using the electrocardiogram input from the cardiac potential measurement means.

(8) In the intracardiac defibrillation catheter system of the present invention, preferably, the power supply device has display means for displaying an electrocardiogram (cardiac potential waveform) input to the arithmetic processing unit, and
the display means displays, together with a cardiac potential waveform indicating the event (Vₙ₊ₘ) sensed after a lapse of the refractory period caused by the event (Vₙ), a mark indicating that it is possible to apply a voltage in synchronization with this cardiac potential waveform, regardless of whether the application execution switch is input.

(9) In the intracardiac defibrillation catheter system of (8) above, the display means preferably displays, together with a cardiac potential waveform indicating the event sensed during the refractory period caused by the event (Vₙ), a mark indicating that it is not possible to apply a voltage in synchronization with this cardiac potential waveform.

### Advantageous Effects of Invention

According to an intracardiac defibrillation catheter system of the present invention, it is possible to reliably avoid performing defibrillation in synchronization with a T wave without being affected by the level of the heart rate of a patient, and it is possible to apply a direct-current voltage to electrodes of a defibrillation catheter to perform defibrillation in synchronization with an R wave of an electrocardiogram input to an arithmetic processing unit.

### Brief Description of Drawings

[Fig. 1] Fig. 1 is a block diagram illustrating an embodiment of an intracardiac defibrillation catheter system of the present invention.
[Fig. 2] Fig. 2 is an explanatory plan view illustrating a fibrillation catheter constituting the catheter system illustrated in Fig. 1.
[Fig. 3] Fig. 3 is an explanatory plan view (a diagram for explaining dimensions and hardness) illustrating the fibrillation catheter constituting the catheter system illustrated in Fig. 1.
[Fig. 4] Fig. 4 is a cross-sectional view illustrating a section A-A in Fig. 2.
[Fig. 5] Fig. 5 includes cross-sectional views illustrating a section B-B, a section C-C, and a section D-D in Fig. 2.
[Fig. 6] Fig. 6 is a perspective view illustrating an internal structure of a handle in an embodiment of the defibrillation catheter illustrated in Fig. 2.
[Fig. 7] Fig. 7 is a partially enlarged view of the inside (distal end side) of the handle illustrated in Fig. 6.
[Fig. 8] Fig. 8 is a partially enlarged view of the inside (proximal end side) of the handle illustrated in Fig. 6.
[Fig. 9] Fig. 9 is an explanatory diagram schematically illustrating a coupled state of a connector of the defibrillation catheter and a catheter connection connector of a power supply device in the catheter system illustrated in Fig. 1.
[Fig. 10] Fig. 10 is a block diagram illustrating the flow of cardiac potential information in a case where a cardiac potential is measured by the defibrillation catheter in the catheter system illustrated in Fig. 1.
[Fig. 11] Fig. 11 is a flowchart (first flowchart) illustrating an example of a process for determining whether defibrillation can be performed in synchronization with each of events sequentially input to an arithmetic processing unit of the power supply device of the catheter system illustrated in Fig. 1.
[Fig. 12] Fig. 12 is an explanatory diagram illustrating an example of the timing of inputting an energy application execution switch and the timing of applying a direct-current voltage in an electrocardiogram input to the arithmetic processing unit of the power supply device of the catheter system illustrated in Fig. 1.
[Fig. 13A] Fig. 13A is the first half of a flowchart (second flowchart) illustrating the action and operation of the power supply device in the catheter system illustrated in Fig. 1.
[Fig. 13B] Fig. 13B is the second half of the flowchart (second flowchart) illustrating the action and operation of the power supply device in the catheter system illustrated in Fig. 1.
[Fig. 14] Fig. 14 is a block diagram illustrating the flow of cardiac potential information in the catheter system illustrated in Fig. 1 in a cardiac potential measurement mode.
[Fig. 15] Fig. 15 is a block diagram illustrating the flow of information related to a measured value of resistance between electrode groups and cardiac potential information in a defibrillation mode of the catheter system illustrated in Fig. 1.
[Fig. 16] Fig. 16 is a block diagram illustrating a state when a direct-current voltage is applied in the defibrillation mode of the catheter system illustrated in Fig. 1.
[Fig. 17] Fig. 17 is a potential waveform diagram measured when the defibrillation catheter constituting the catheter system illustrated in Fig. 1 applies predetermined electrical energy.
[Fig. 18] Fig. 18 is an explanatory diagram illustrating an example of a screen of a display means of the power supply device of the catheter system illustrated in Fig. 1.

### Description of Embodiments

An embodiment of the present invention will be described hereinafter.

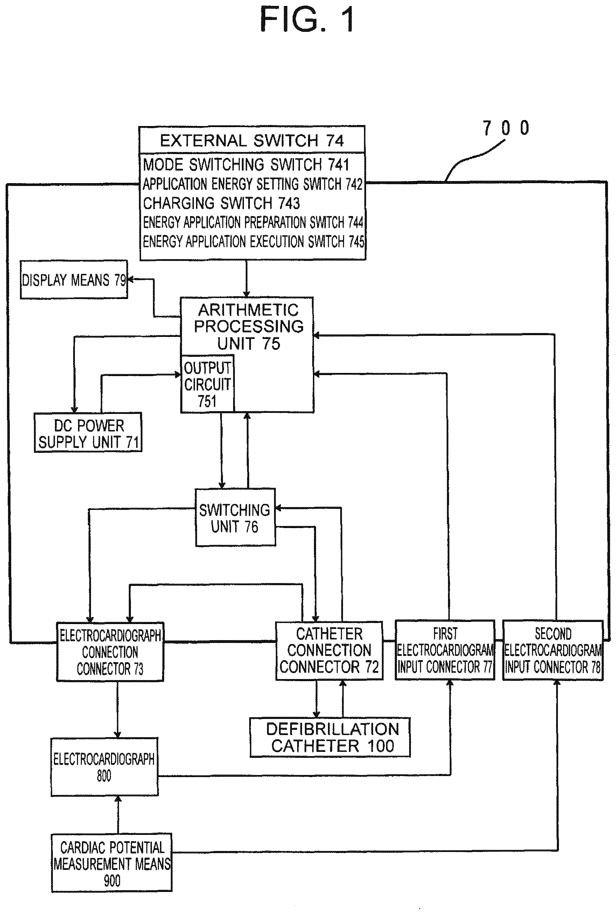

As illustrated in Fig. 1, an intracardiac defibrillation catheter system of this embodiment includes a defibrillation catheter 100, a power supply device 700, an electrocardiograph 800, and a cardiac potential measurement means 900.

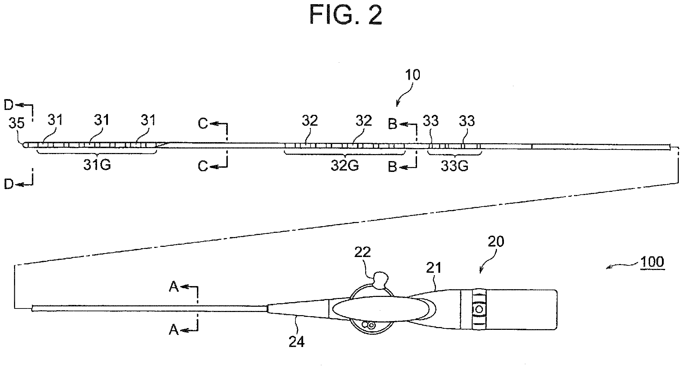

As illustrated in Fig. 2 to Fig. 5, the defibrillation catheter 100 constituting the defibrillation catheter system of this embodiment includes a multi-lumen tube 10, a handle 20, a first DC electrode group 31G, a second DC electrode group 32G, a proximal-end-side potential measurement electrode group 33G, a first lead wire group 41G, a second lead wire group 42G, and a third lead wire group 43G.

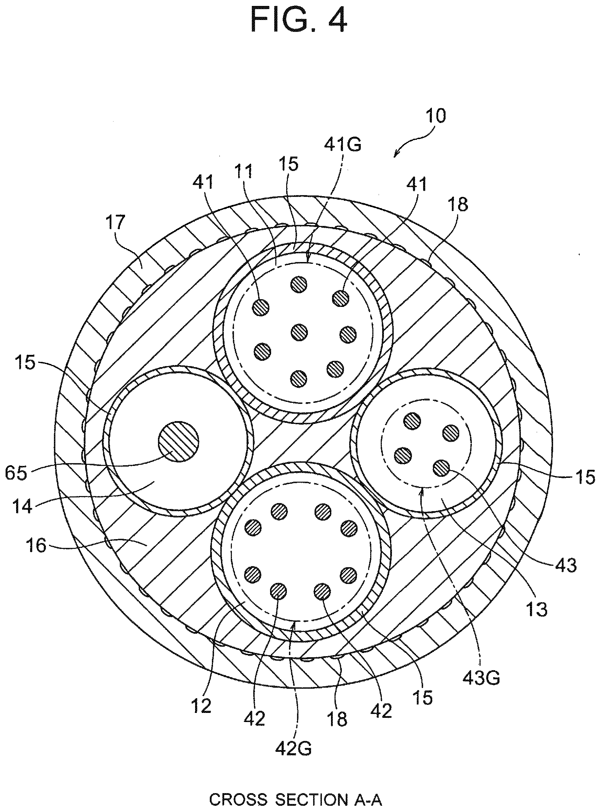

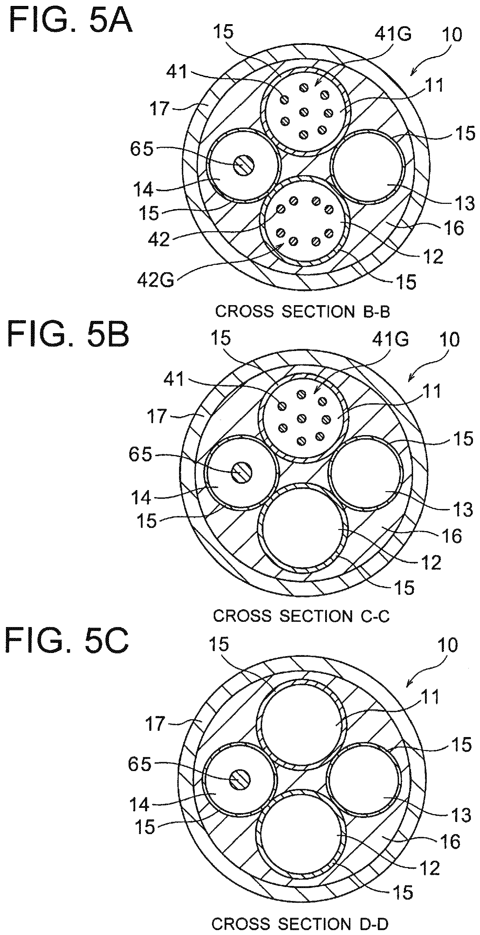

As illustrated in Fig. 4 and Fig. 5, the multi-lumen tube 10 (an insulating tube member having a multi-lumen structure) constituting the defibrillation catheter 100 has formed therein four lumens (a first lumen 11, a second lumen 12, a third lumen 13, and a fourth lumen 14).

In Fig. 4 and Fig. 5, reference numeral 15 denotes fluororesin layers defining the lumens, reference numeral 16 denotes an inner (core) portion composed of a nylon elastomer having low hardness, and reference numeral 17 denotes an outer (shell) portion composed of a nylon elastomer having high hardness. In Fig. 4, reference numeral 18 denotes an individual stainless steel wire forming a braid.

The fluororesin layers 15 defining the lumens are made of, for example, a material having high insulating properties, such as perfluoroalkyl vinyl ether copolymer (PFA) or polytetrafluoroethylene (PTFE).

The nylon elastomer constituting the outer portion 17 of the multi-lumen tube 10, having different hardnesses in an axial direction, is used. Accordingly, the multi-lumen tube 10 is configured such that the hardness increases stepwise from the distal end side toward the proximal end side.

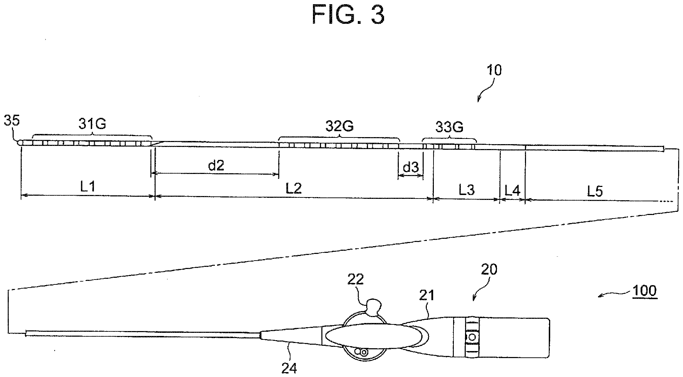

As a preferred example, in Fig. 3, the hardness (hardness obtained by a D-type hardness meter) of a region indicated by L1 (a length of 52 mm) is 40, the hardness of a region indicated by L2 (a length of 108 mm) is 55, the hardness of a region indicated by L3 (a length of 25.7 mm) is 63, the hardness of a region indicated by L4 (a length of 10 mm) is 68, and the hardness of a region indicated by L5 (a length of 500 mm) is 72.

The braid constituted by the individual stainless steel wire 18 is formed only in the region indicated by L5 in Fig. 3 and is disposed between, as illustrated in Fig. 4, the inner portion 16 and the outer portion 17.

The outer diameter of the multi-lumen tube 10 is set to be, for example, 1.2 to 3.3 mm.

A method for manufacturing the multi-lumen tube 10 is not limited in particular.

The handle 20 constituting the defibrillation catheter 100 in this embodiment includes a handle body 21, a knob 22, and a strain relief 24.

The knob 22 is operated to rotate, thereby enabling a distal end portion of the multi-lumen tube 10 to be deflected (oscillated).

The first DC electrode group 31G, the second DC electrode group 32G, and the proximal-end-side potential measurement electrode group 33G are mounted in an outer periphery (distal end region having no braiding formed on the inside thereof) of the multi-lumen tube 10. Here, the "electrode group" refers to an assembly of a plurality of electrodes mounted at narrow intervals (for example, less than or equal to 5 mm) and constituting the same pole (having the same polarity) or having the same purpose.

The first DC electrode group is configured such that a plurality of electrodes that is to constitute the same pole (the minus pole or the plus pole) are mounted at narrow intervals in the distal end region of the multi-lumen tube. Here, the number of electrodes constituting the first DC electrode group, which differs depending on the width or arrangement interval of the electrodes, is set to be, for example, 4 to 13, and is preferably set to be 8 to 10.

In this embodiment, the first DC electrode group 31G is constituted by eight ring-shaped electrodes 31 mounted in the distal end region of the multi-lumen tube 10.

The electrodes 31 constituting the first DC electrode group 31G are connected to a catheter connection connector of the power supply device 700 via lead wires (lead wires 41 constituting the first lead wire group 41G) and a connector described below.

Here, the width (length in the axial direction) of the electrodes 31 is preferably 2 to 5 mm, and is 4 mm as a preferred example.

If the width of the electrodes 31 is too narrow, the amount of heat generated when a voltage is applied becomes excessively large, and the surrounding tissues may be damaged. If the width of the electrodes 31 is too wide, in contrast, the flexibility and stretchability of a portion of the multi-lumen tube 10 where the first DC electrode group 31G is disposed may be impaired.

The mounting interval of the electrodes 31 (separation distance between adjacent electrodes) is preferably 1 to 5 mm, and is 2 mm as a preferred example.

When the defibrillation catheter 100 is in use (arranged in a cardiac cavity), the first DC electrode group 31G is positioned in a coronary sinus, for example.

The second DC electrode group is configured such that a plurality of electrodes that is to constitute a pole (the plus pole or the minus pole) opposite to that of the first DC electrode group are mounted at narrow intervals at positions spaced away toward the proximal end side from the position at which the first DC electrode group of the multi-lumen tube is mounted. Here, the number of electrodes constituting the second DC electrode group, which differs depending on the width or arrangement interval of the electrodes, is set to be, for example, 4 to 13, and is preferably set to be 8 to 10.

In this embodiment, the second DC electrode group 32G is constituted by eight ring-shaped electrodes 32 mounted on the multi-lumen tube 10 at positions spaced away toward the proximal end side from the position at which the first DC electrode group 31G is mounted.

The electrodes 32 constituting the second DC electrode group 32G are connected to the catheter connection connector of the power supply device 700 via lead wires (lead wires 42 constituting the second lead wire group 42G) and a connector described below.

Here, the width (length in the axial direction) of the electrodes 32 is preferably 2 to 5 mm, and is 4 mm as a preferred example.

If the width of the electrodes 32 is too narrow, the amount of heat generated when a voltage is applied becomes excessively large, and the surrounding tissues may be damaged. If the width of the electrodes 32 is too wide, in contrast, the flexibility and stretchability of a portion of the multi-lumen tube 10 where the second DC electrode group 32G is disposed may be impaired.

The mounting interval of the electrodes 32 (separation distance between adjacent electrodes) is preferably 1 to 5 mm, and is 2 mm as a preferred example.

When the defibrillation catheter 100 is in use (arranged in a cardiac cavity), the second DC electrode group 32G is positioned in a right atrium, for example.

In this embodiment, the proximal-end-side potential measurement electrode group 33G is constituted by four ring-shaped electrodes 33 mounted on the multi-lumen tube 10 at positions spaced away toward the proximal end side from the position at which the second DC electrode group 32G is mounted.

The electrodes 33 constituting the proximal-end-side potential measurement electrode group 33G are connected to the catheter connection connector of the power supply device 700 via lead wires (lead wires 43 constituting the third lead wire group 43G) and a connector described below.

Here, the width (length in the axial direction) of the electrodes 33 is preferably 0.5 to 2.0 mm, and is 1.2 mm as a preferred example.

If the width of the electrodes 33 is too wide, the measurement accuracy of the cardiac potential may be reduced, or an area where an abnormal potential occurs may be difficult to identify.

The mounting interval of the electrodes 33 (separation distance between adjacent electrodes) is preferably 1.0 to 10.0 mm, and is 5 mm as a preferred example.

When the defibrillation catheter 100 is in use (arranged in a cardiac cavity), the proximal-end-side potential measurement electrode group 33G is positioned in a superior vena cava where an abnormal potential is likely to occur, for example.

The defibrillation catheter 100 has a distal end tip 35 mounted at a distal end thereof.

The distal end tip 35 is not connected to a lead wire and is not used as an electrode in this embodiment. However, by connecting the distal end tip 35 to a lead wire, the distal end tip 35 can be used as an electrode. The distal end tip 35 is made of a material, non-limiting examples of which include metal materials such as platinum and stainless steel, and various resin materials.

A separation distance d2 between the first DC electrode group 31G (the electrode 31 on the proximal end side) and the second DC electrode group 32G (the electrode 32 on the distal end side) is preferably 40 to 100 mm, and is 66 mm as a preferred example.

A separation distance d3 between the second DC electrode group 32G (the electrode 32 on the proximal end side) and the proximal-end-side potential measurement electrode group 33G (the electrode 33 on the distal end side) is preferably 5 to 50 mm, and is 30 mm as a preferred example.

The electrodes 31, 32, and 33 constituting the first DC electrode group 31G, the second DC electrode group 32G, and the proximal-end-side potential measurement electrode group 33G are preferably composed of platinum or a platinum-based alloy to provide good imaging contrast for X-rays.

The first lead wire group 41G illustrated in Fig. 4 and Fig. 5 is an assembly of eight lead wires 41 respectively connected to the eight electrodes (31) constituting the first DC electrode group (31G).

The first lead wire group 41G (the lead wires 41) can electrically connect each of the eight electrodes 31 constituting the first DC electrode group 31G to the power supply device 700.

The eight electrodes 31 constituting the first DC electrode group 31G are connected to the respective different lead wires 41. Each of the lead wires 41 is welded, at its distal end portion, to an inner peripheral surface of a corresponding one of the electrodes 31 and enters the first lumen 11 through a side hole formed in a tube wall of the multi-lumen tube 10. The eight lead wires 41, which enter the first lumen 11, extend through the first lumen 11 as the first lead wire group 41G.

The second lead wire group 42G illustrated in Fig. 4 and Fig. 5 is an assembly of eight lead wires 42 respectively connected to the eight electrodes (32) constituting the second DC electrode group (32G).

The second lead wire group 42G (the lead wires 42) can electrically connect each of the eight electrodes 32 constituting the second DC electrode group 32G to the power supply device 700.

The eight electrodes 32 constituting the second DC electrode group 32G are connected to the respective different lead wires 42. Each of the lead wires 42 is welded, at its distal end portion, to an inner peripheral surface of a corresponding one of the electrodes 32 and enters the second lumen 12 (a lumen different from the first lumen 11 through which the first lead wire group 41G extends) through a side hole formed in the tube wall of the multi-lumen tube 10. The eight lead wires 42, which enter the second lumen 12, extend through the second lumen 12 as the second lead wire group 42G.

As described above, the first lead wire group 41G extends through the first lumen 11, and the second lead wire group 42G extends through the second lumen 12, thereby allowing both lead wire groups to be completely insulated and isolated from each other in the multi-lumen tube 10. This can reliably prevent short circuit between the first lead wire group 41G (the first DC electrode group 31G) and the second lead wire group 42G (the second DC electrode group 32G) when a voltage necessary for defibrillation is applied.

The third lead wire group 43G illustrated in Fig. 4 is an assembly of four lead wires 43 respectively connected to the electrodes (33) constituting the proximal-end-side potential measurement electrode group (33G).

The third lead wire group 43G (the lead wires 43) can electrically connect each of the electrodes 33 constituting the proximal-end-side potential measurement electrode group 33G to the power supply device 700.

The four electrodes 33 constituting the proximal-end-side potential measurement electrode group 33G are connected to the respective different lead wires 43. Each of the lead wires 43 is welded, at its distal end portion, to an inner peripheral surface of a corresponding one of the electrodes 33 and enters the third lumen 13 through a side hole formed in the tube wall of the multi-lumen tube 10. The four lead wires 43, which enter the third lumen 13, extend through the third lumen 13 as the third lead wire group 43G.

As described above, the third lead wire group 43G, which extends through the third lumen 13, is completely insulated and isolated from both the first lead wire group 41G and the second lead wire group 42G. This can reliably prevent short circuit between the third lead wire group 43G (the proximal-end-side potential measurement electrode group 33G) and the first lead wire group 41G (the first DC electrode group 31G) or the second lead wire group 42G (the second DC electrode group 32G) when a voltage necessary for defibrillation is applied.

All of the lead wires 41, the lead wires 42, and the lead wires 43 are composed of a resin-coating wire obtained by coating an outer peripheral surface of a metal conducting wire with a resin such as polyimide. Here, the film thickness of the coating resin is set to be about 2 to 30 µm.

In Fig. 4 and Fig. 5, reference numeral 65 denotes a pull wire.

The pull wire 65 extends through the fourth lumen 14 and extends eccentrically from the center axis of the multi-lumen tube 10.

A distal end portion of the pull wire 65 is fixed to the distal end tip 35 using solder. Furthermore, a large-diameter portion for preventing fall-off (fall-off preventing portion) may be formed at a distal end of the pull wire 65. Accordingly, the distal end tip 35 and the pull wire 65 are firmly bonded to each other, and the distal end tip 35 can be reliably prevented from falling or the like.

On the other hand, a proximal end portion of the pull wire 65 is connected to the knob 22 of the handle 20, and the knob 22 is operated to pull the pull wire 65, thereby allowing the distal end portion of the multi-lumen tube 10 to be deflected.

The pull wire 65 is made of stainless steel or a Ni-Ti-based superelastic alloy. However, the pull wire 65 may not necessarily be made of a metal. The pull wire 65 may be constituted by, for example, a non-conducting wire or the like having high strength.

Note that a mechanism for deflecting the distal end portion of the multi-lumen tube is not limited to this and may include a leaf spring, for example.

Only the pull wire 65 extends through the fourth lumen 14 in the multi-lumen tube 10, and no lead wire (group) extends through the fourth lumen 14. Accordingly, during the operation of deflecting the distal end portion of the multi-lumen tube 10, the pull wire 65 moving in the axial direction can be prevented from damaging (for example, abrading) lead wires.

In the defibrillation catheter 100 in this embodiment, also inside the handle 20, the first lead wire group 41G, the second lead wire group 42G, and the third lead wire group 43G are insulated and isolated from each other.

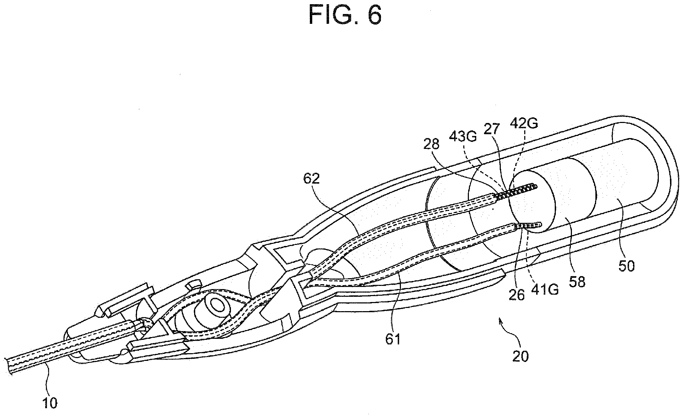

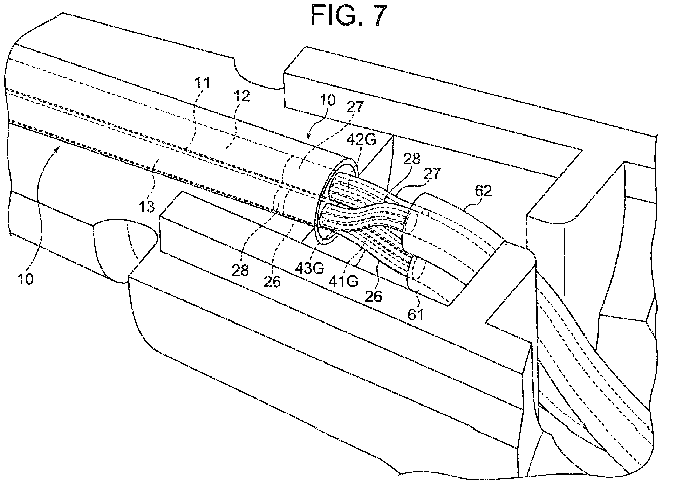

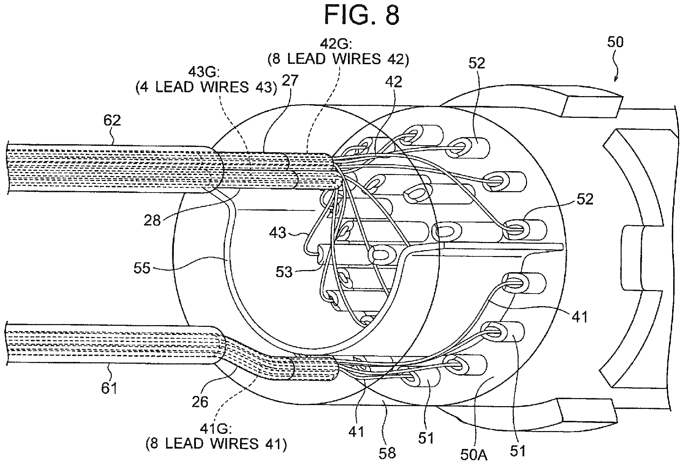

Fig. 6 is a perspective view illustrating an internal structure of the handle of the defibrillation catheter 100 in this embodiment, Fig. 7 is a partially enlarged view of the inside (distal end side) of the handle, and Fig. 8 is a partially enlarged view of the inside (proximal end side) of the handle.

As illustrated in Fig. 6, a proximal end portion of the multi-lumen tube 10 is inserted into a distal end opening in the handle 20, thereby allowing the multi-lumen tube 10 and the handle 20 to be connected to each other.

As illustrated in Fig. 6 and Fig. 8, a cylindrical connector 50 configured such that a plurality of pin terminals (51, 52, and 53) projecting in a distal end direction are arranged on a distal end surface 50A is built in a proximal end portion of the handle 20.

Furthermore, as illustrated in Fig. 6 to Fig. 8, three insulating tubes (a first insulating tube 26, a second insulating tube 27, and a third insulating tube 28) through which the three lead wire groups (the first lead wire group 41G, the second lead wire group 42G, and the third lead wire group 43G) are to be respectively inserted extend through the inside of the handle 20.

As illustrated in Fig. 6 and Fig. 7, a distal end portion (a portion that is about 10 mm from the distal end) of the first insulating tube 26 is inserted into the first lumen 11 in the multi-lumen tube 10, thereby allowing the first insulating tube 26 to be coupled to the first lumen 11 through which the first lead wire group 41G extends.

The first insulating tube 26 coupled to the first lumen 11 passes through an inner hole of a first protection tube 61 extending through the inside of the handle 20 and extends up to the vicinity of the connector 50 (the distal end surface 50A where the pin terminals are arranged) to form an insertion path through which a proximal end portion of the first lead wire group 41G is guided to the vicinity of the connector 50. Accordingly, the first lead wire group 41G extending out of the multi-lumen tube 10 (the first lumen 11) can extend through the inside of the handle 20 (the inner hole of the first insulating tube 26) without kinking.

The first lead wire group 41G extending out of the proximal end opening of the first insulating tube 26 is separated into the eight lead wires 41 constituting the first lead wire group 41G, and each of these lead wires 41 is connected and fixed to one of the pin terminals arranged on the distal end surface 50A of the connector 50 using solder. Here, a region where the pin terminals (the pin terminals 51) to which the lead wires 41 constituting the first lead wire group 41G are connected and fixed are arranged is referred to as a "first terminal group region".

A distal end portion (a portion that is about 10 mm from the distal end) of the second insulating tube 27 is inserted into the second lumen 12 in the multi-lumen tube 10, thereby allowing the second insulating tube 27 to be coupled to the second lumen 12 through which the second lead wire group 42G extends.

The second insulating tube 27 coupled to the second lumen 12 passes through an inner hole of a second protection tube 62 extending through the inside of the handle 20 and extends up to the vicinity of the connector 50 (the distal end surface 50A where the pin terminals are arranged) to form an insertion path through which a proximal end portion of the second lead wire group 42G is guided to the vicinity of the connector 50. Accordingly, the second lead wire group 42G extending out of the multi-lumen tube 10 (the second lumen 12) can extend through the inside of the handle 20 (the inner hole of the second insulating tube 27) without kinking.

The second lead wire group 42G extending out of the proximal end opening of the second insulating tube 27 is separated into the eight lead wires 42 constituting the second lead wire group 42G, and each of these lead wires 42 is connected and fixed to one of the pin terminals arranged on the distal end surface 50A of the connector 50 using solder. Here, a region where the pin terminals (the pin terminals 52) to which the lead wires 42 constituting the second lead wire group 42G are connected and fixed are arranged is referred to as a "second terminal group region".

A distal end portion (a portion that is about 10 mm from the distal end) of the third insulating tube 28 is inserted into the third lumen 13 in the multi-lumen tube 10, thereby allowing the third insulating tube 28 to be coupled to the third lumen 13 through which the third lead wire group 43G extends.

The third insulating tube 28 coupled to the third lumen 13 passes through the inner hole of the second protection tube 62 extending through the inside of the handle 20 and extends up to the vicinity of the connector 50 (the distal end surface 50A where the pin terminals are arranged) to form an insertion path through which a proximal end portion of the third lead wire group 43G is guided to the vicinity of the connector 50. Accordingly, the third lead wire group 43G extending out of the multi-lumen tube 10 (the third lumen 13) can extend through the inside of the handle 20 (the inner hole of the third insulating tube 28) without kinking.

The third lead wire group 43G extending out of the proximal end opening of the third insulating tube 28 is separated into the four lead wires 43 constituting the third lead wire group 43G, and each of these lead wires 43 is connected and fixed to one of the pin terminals arranged on the distal end surface 50A of the connector 50 using solder. Here, a region where the pin terminals (the pin terminals 53) to which the lead wires 43 constituting the third lead wire group 43G are connected and fixed are arranged is referred to as a "third terminal group region".

Here, the insulating tubes (the first insulating tube 26, the second insulating tube 27, and the third insulating tube 28) are made of a material, examples of which can include polyimide resins, polyamide resins, and polyamideimide resins. Among them, the polyimide resins, which are high in hardness, easy to insert a lead wire group therethrough, and capable of forming a thin-walled component, are particularly preferable.

The wall thickness of the insulating tubes is preferably 20 to 40 µm, and is 30 µm as a preferred example.

Furthermore, examples of the material of the protection tubes (the first protection tube 61 and the second protection tube 62) into which the insulating tubes are to be inserted can include nylon-based elastomers such as "Pebax" (a registered trademark of ARKEMA).

According to the defibrillation catheter 100 in this embodiment having the configuration described above, the first lead wire group 41G extends in the first insulating tube 26, the second lead wire group 42G extends in the second insulating tube 27, and the third lead wire group 43G extends in the third insulating tube 28. Thus, the first lead wire group 41G, the second lead wire group 42G, and the third lead wire group 43G can be completely insulated and isolated from each other even inside the handle 20. As a result, short circuit among the first lead wire group 41G, the second lead wire group 42G, and the third lead wire group 43G inside the handle 20 (in particular, short circuit among lead wire groups extending out around the openings of the lumens) can be reliably prevented when a voltage necessary for defibrillation is applied.

In addition, inside the handle 20, the first insulating tube 26 is protected by the first protection tube 61, and the second insulating tube 27 and the third insulating tube 28 are protected by the second protection tube 52, whereby, for example, the insulating tubes can be prevented from being damaged by a constituent member (movable component) of the knob 22 coming into contact or rub thereagainst during the operation of deflecting the distal end portion of the multi-lumen tube 10.

The defibrillation catheter 100 in this embodiment includes a partition plate 55 for separating, on the distal end surface 50A of the connector 50 where the plurality of pin terminals are arranged, the first terminal group region from the second terminal group region and the third terminal group region and isolating the lead wires 41 from the lead wires 42 and the lead wires 43.

The partition plate 55, which separates the first terminal group region from the second terminal group region and the third terminal group region, is configured by molding an insulating resin into a gutter shape having a flat surface on both sides thereof. The insulating resin constituting the partition plate 55 is not limited in particular, and a general-purpose resin such as polyethylene can be used.

The thickness of the partition plate 55 is set to be, for example, 0.1 to 0.5 mm, and is 0.2 mm as a preferred example.

The height (distance from a proximal end edge to a distal end edge) of the partition plate 55 is required to be larger than a separation distance between the distal end surface 50A of the connector 50 and the insulating tubes (the first insulating tube 26 and the second insulating tube 27). When the separation distance is 7 mm, the height of the partition plate 55 is set to be, for example, 8 mm. A partition plate having a height less than 7 mm does not enable its distal end edge to be positioned more toward the distal end side than the proximal ends of the insulating tubes.

According to such a configuration, the lead wires 41 constituting the first lead wire group 41G (proximal end portions of the lead wires 41 extending out of the proximal end opening of the first insulating tube 26), and the lead wires 42 constituting the second lead wire group 42G (proximal end portions of the lead wires 42 extending out of the proximal end opening of the second insulating tube 27) can be reliably and systematically isolated from each other.

If the partition plate 55 is not included, it is not possible to systematically isolate (separate) the lead wires 41 and the lead wires 42 from each other, and these wires may be crossed.

Further, since the lead wires 41 constituting the first lead wire group 41G and the lead wires 42 constituting the second lead wire group 42G, to which voltages having different polarities are applied, are isolated from each other and do not come into contact with each other by the partition plate 55, even if a voltage necessary for intracardiac defibrillation is applied when the defibrillation catheter 100 is in use, no short circuit occurs between the lead wires 41 constituting the first lead wire group 41G (the proximal end portions of the lead wires 41 extending out of the proximal end opening of the first insulating tube 26) and the lead wires 42 constituting the second lead wire group 42G (the proximal end portions of the lead wires 42 extending out of the proximal end opening of the second insulating tube 27).

Furthermore, if an error occurs when the lead wires are connected and fixed to the pin terminals during manufacturing of the defibrillation catheter, for example, if the lead wires 41 constituting the first lead wire group 41G are connected to the pin terminals in the second terminal group region, these leads 41 cross the partition 55. Thus, the error in connection can be easily found.

Note that the lead wires 43 (the pin terminals 53) constituting the third lead wire group 43G, together with the lead wires 42 (the pin terminals 52), are isolated from the lead wires 41 (the pin terminals 51) by the partition plate 55; however, this is not limiting, and the lead wires 43 (the pin terminals 53), together with the lead wires 41 (the pin terminals 51), may be isolated from the lead wires 42 (the pin terminals 52) by the partition plate 55.

In the defibrillation catheter 100, the distal end edge of the partition plate 55 is located more toward the distal end side than any of the proximal end of the first insulating tube 26 and the proximal end of the second insulating tube 27.

Accordingly, the partition plate 55 is always present between the lead wires extending out of the proximal end opening of the first insulating tube 26 (the lead wires 41 constituting the first lead wire group 41G) and the lead wires extending out of the proximal end opening of the second insulating tube 27 (the lead wires 42 constituting the second lead wire group 42G), and short circuit caused by contact between the lead wires 41 and the lead wires 42 can be reliably prevented.

As illustrated in Fig. 8, the eight lead wires 41 extending out of the proximal end opening of the first insulating tube 26 and connected and fixed to the pin terminals 51 of the connector 50, the eight lead wires 42 extending out of the proximal end opening of the second insulating tube 27 and connected and fixed to the pin terminals 52 of the connector 50, and the four lead wires 43 extending out of the proximal end opening of the third insulating tube 28 and connected and fixed to the pin terminals 53 of the connector 50 are covered at peripheries thereof by a resin 58, thereby allowing the respective shapes to be maintained and fixed.

The resin 58 for maintaining the shapes of the lead wires is formed into a cylindrical shape having the same diameter as the connector 50, and the pin terminals, the lead wires, the proximal end portions of the insulating tubes, and the partition plate 55 are embedded in the inside of this resin-formed body.

Further, according to the configuration in which the proximal end portions of the insulating tubes are embedded in the inside of the resin-formed body, the entire area of the lead wires (proximal end portions) extending out of the proximal end openings of the insulating tubes and connected and fixed to the pin terminals can be completely covered with the resin 58, and the shapes of the lead wires (proximal end portions) can be completely maintained and fixed.

Furthermore, the height (distance from a proximal end surface to a distal end surface) of the resin-formed body is preferably larger than the height of the partition plate 55, and is set to, for example, 9 mm when the height of the partition plate 55 is 8 mm.

Here, the resin 58 constituting the resin-formed body is not limited in particular. However, a thermosetting resin or a photo-setting resin is preferably used. Specific examples can include urethane-based, epoxy-based, and urethane-epoxy-based setting resins.

According to such a configuration as described above, since the shapes of the lead wires are maintained and fixed by the resin 58, it is possible to prevent the lead wires extending out of the proximal end openings of the insulating tubes from being damaged (for example, the coating resins of the lead wires from being cracked) by kinking or coming into contact with edges of the pin terminals during manufacturing of the defibrillation catheter 100 (when the connector 50 is mounted on the inside of the handle 20).

As illustrated in Fig. 1, the power supply device 700 constituting the defibrillation catheter system of this embodiment includes a DC power supply unit 71, a catheter connection connector 72, an electrocardiograph connection connector 73, an external switch (input means) 74, an arithmetic processing unit 75, a switching unit 76, a first electrocardiogram input connector 77, a second electrocardiogram input connector 78, and a display means 79.

The DC power supply unit 71 has a capacitor built therein, and the built-in capacitor is charged in response to an input of the external switch 74 (a charging switch 743).

The catheter connection connector 72 is connected to the connector 50 of the defibrillation catheter 100, and is electrically connected to the proximal end side of the first lead wire group (41G), the second lead wire group (42G), and the third lead wire group (43G).

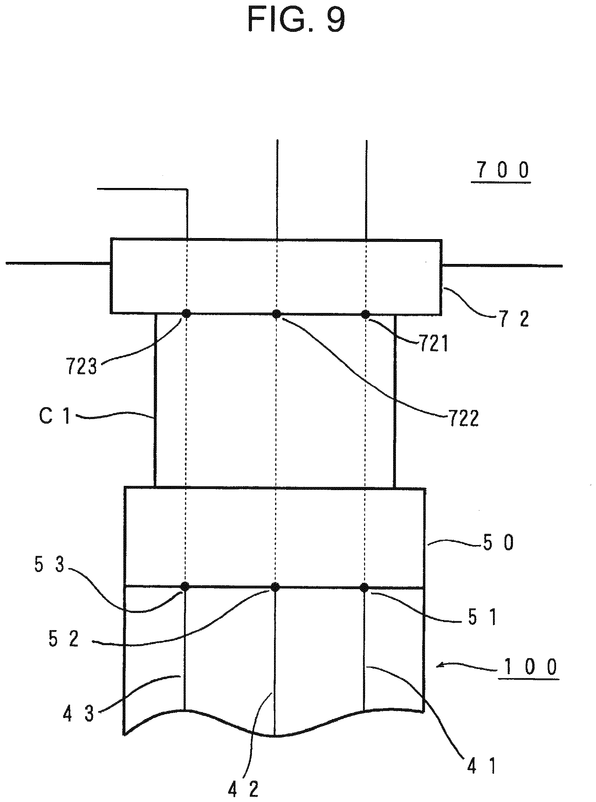

As illustrated in Fig. 9, the connector 50 of the defibrillation catheter 100 and the catheter connection connector 72 of the power supply device 700 are coupled to each other by a connector cable C1, whereby
the pin terminals 51 (actually, eight) to which the eight lead wires 41 constituting the first lead wire group are connected and fixed are connected to terminals 721 (actually, eight) of the catheter connection connector 72,
the pin terminals 52 (actually, eight) to which the eight lead wires 42 constituting the second lead wire group are connected and fixed are connected to terminals 722 (actually, eight) of the catheter connection connector 72, and
the pin terminals 53 (actually, four) to which the four lead wires 43 constituting the third lead wire group are connected and fixed are connected to terminals 723 (actually, four) of the catheter connection connector 72.

Here, the terminals 721 and the terminals 722 of the catheter connection connector 72 are connected to the switching unit 76, and the terminals 723 are directly connected to the electrocardiograph connection connector 73 without the intervention of the switching unit 76.

Accordingly, cardiac potential information measured by the first DC electrode group 31G and the second DC electrode group 32G reaches the electrocardiograph connection connector 73 through the switching unit 76, and cardiac potential information measured by the proximal-end-side potential measurement electrode group 33G reaches the electrocardiograph connection connector 73 without the intervention of the switching unit 76.

The electrocardiograph connection connector 73 is connected to an input terminal of the electrocardiograph 800.

The external switch 74, which is an input means, is composed of a mode switching switch 741 for switching between a cardiac potential measurement mode and a defibrillation mode, an application energy setting switch 742 that sets electrical energy to be applied for defibrillation, a charging switch 743 for charging the DC power supply unit 71,, an energy application preparation switch 744 for preparing defibrillation (switching the contact of the switching unit 76 described below), and an energy application execution switch (discharging switch) 745 that is input after (or at the same time as when) the energy application preparation switch 744 is input to apply electrical energy to execute defibrillation. All input signals from these external switches 74 are sent to the arithmetic processing unit 75.

Since the energy application preparation switch 744 is included as a switch for applying energy in addition to the energy application execution switch 745, the user is able to check the state of the cardiac potential waveform before inputting the energy application execution switch 745.

Accordingly, if a disturbance (such as drift or noise, for example) of the cardiac potential waveform occurs when the energy application preparation switch 744 is input and the contact of the switching unit 76 is switched to a second contact, the execution of the application of energy can be avoided.

The arithmetic processing unit 75 controls the DC power supply unit 71, the switching unit 76, and the display means 79 on the basis of an input of the external switch 74.

The arithmetic processing unit 75 has an output circuit 751 for outputting a direct-current voltage from the DC power supply unit 71 to electrodes of the defibrillation catheter 100 via the switching unit 76.

With the output circuit 751, direct-current voltages can be applied so that the terminals 721 of the catheter connection connector 72 illustrated in Fig. 9 (finally, the first DC electrode group 31G of the defibrillation catheter 100) and the terminals 722 of the catheter connection connector 72 (finally, the second DC electrode group 32G of the defibrillation catheter 100) have different polarities (when one electrode group becomes a minus pole, the other electrode group becomes a plus pole).

The switching unit 76 is composed of a one-circuit two-contact (Single Pole Double Throw) switching switch having a common contact to which the catheter connection connector 72 (the terminals 721 and the terminals 722) is connected, a first contact to which the electrocardiograph connection connector 73 is connected, and a second contact to which the arithmetic processing unit 75 is connected.

That is, when the first contact is selected (when the first contact is connected to the common contact), a path connecting the catheter connection connector 72 and the electrocardiograph connection connector 73 to each other is ensured, and when the second contact is selected (when the second contact is connected to the common contact), a path connecting the catheter connection connector 72 and the arithmetic processing unit 75 to each other is ensured.

The switching operation of the switching unit 76 is controlled by the arithmetic processing unit 75 on the basis of an input of the external switch 74 (the mode switching switch 741/the energy application preparation switch 744).

The first electrocardiogram input connector 77 is connected to the arithmetic processing unit 75 and is furthermore connected to an output terminal of the electrocardiograph 800.

With the first electrocardiogram input connector 77, cardiac potential information (electrocardiogram) output from the electrocardiograph 800 can be input to the arithmetic processing unit 75.

The second electrocardiogram input connector 78 is connected to the arithmetic processing unit 75 and is furthermore connected to an output terminal of the cardiac potential measurement means 900.

Here, examples of the cardiac potential measurement means 900 can include an electrode pad to be attached to the surface of the body of a patient to measure a 12-lead electrocardiogram, and an electrode catheter to be placed into a heart of a patient (an electrode catheter different from the defibrillation catheter 100).

With the second electrocardiogram input connector 78, cardiac potential information (electrocardiogram) output from the cardiac potential measurement means 900 can be input to the arithmetic processing unit 75.

The arithmetic processing unit 75 can control the DC power supply unit 71 and the switching unit 76 on the basis of the cardiac potential information input through the first electrocardiogram input connector 77 or the second electrocardiogram input connector 78.

The display means 79 is connected to the arithmetic processing unit 75, and the cardiac potential information (electrocardiogram) input to the arithmetic processing unit 75 from the first electrocardiogram input connector 77 or the second electrocardiogram input connector 78 is displayed on the display means 79 to allow the operator to perform defibrillation treatment (such as an input of the external switch) while monitoring the cardiac potential information (electrocardiogram) input to the arithmetic processing unit 75.

The electrocardiograph 800 (input terminal) constituting the defibrillation catheter system of this embodiment is connected to the electrocardiograph connection connector 73 of the power supply device 700, and cardiac potential information measured by the defibrillation catheter 100 (the electrodes constituting the first DC electrode group 31G, the second DC electrode group 32G, and the proximal-end-side potential measurement electrode group 33G) is input from the electrocardiograph connection connector 73 to the electrocardiograph 800.

Furthermore, the electrocardiograph 800 (another input terminal) is also connected to the cardiac potential measurement means 900, and cardiac potential information measured by the cardiac potential measurement means 900 is also input to the electrocardiograph 800.

The electrocardiograph 800 (output terminal) is connected to the first electrocardiogram input connector 77 of the power supply device 700 and can send a portion of the cardiac potential information input to the electrocardiograph 800 (the cardiac potential information from the defibrillation catheter 100 and the cardiac potential information from the cardiac potential measurement means 900) to the arithmetic processing unit 75 through the first electrocardiogram input connector 77.

The defibrillation catheter 100 in this embodiment can be used as a cardiac potential measurement electrode catheter when defibrillation treatment is not required.

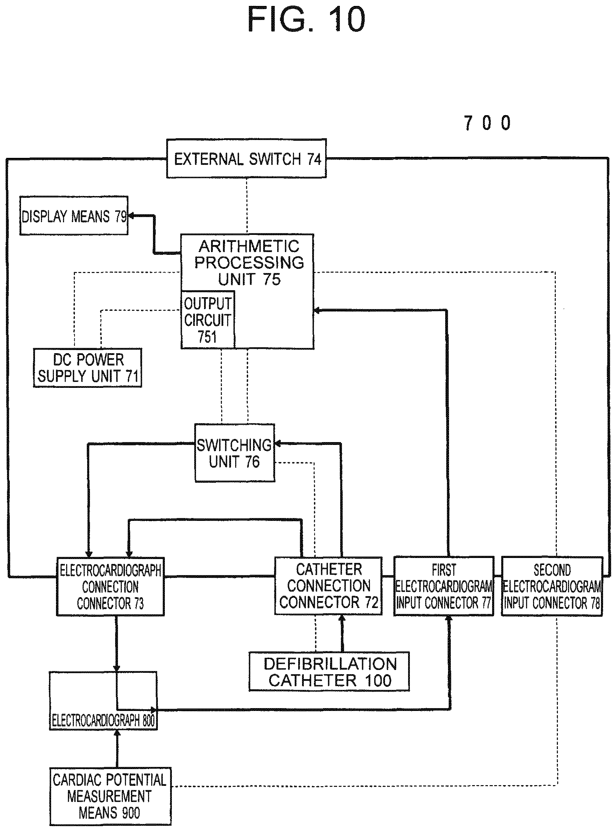

Fig. 10 illustrates the flow of cardiac potential information in a case where the defibrillation catheter 100 according to this embodiment measures a cardiac potential when performing cardiac catheterization (for example, highfrequency treatment).

At this time, the switching unit 76 of the power supply device 700 selects the first contact to which the electrocardiograph connection connector 73 is connected.

The cardiac potential measured by the electrodes constituting the first DC electrode group 31G and/or the second DC electrode group 32G of the defibrillation catheter 100 is input to the electrocardiograph 800 through the catheter connection connector 72, the switching unit 76, and the electrocardiograph connection connector 73.

Furthermore, the cardiac potential measured by the electrodes constituting the proximal-end-side potential measurement electrode group 33G of the defibrillation catheter 100 is input to the electrocardiograph 800 from the catheter connection connector 72 directly through the electrocardiograph connection connector 73 without passing through the switching unit 76.

The cardiac potential information from the defibrillation catheter 100 (electrocardiogram) is displayed on a monitor (not illustrated) of the electrocardiograph 800.

Furthermore, a portion of the cardiac potential information from the defibrillation catheter 100 (for example, a potential difference between the electrodes 31 constituting the first DC electrode group 31G (a first pole and a second pole)) can be input from the electrocardiograph 800 to the display means 79 through the first electrocardiogram input connector 77 and the arithmetic processing unit 75 and displayed on the display means 79.

As described above, the defibrillation catheter 100 can be used as a cardiac potential measurement electrode catheter when defibrillation treatment is not required during cardiac catheterization.

Further, when atrial fibrillation has occurred during cardiac catheterization, the defibrillation catheter 100, which has been used as an electrode catheter, can be used to immediately perform defibrillation treatment. As a result, when atrial fibrillation has occurred, efforts, such as inserting a new catheter for defibrillation, can be saved.

The arithmetic processing unit 75 sequentially senses, from the cardiac potential information (electrocardiogram) sent from the electrocardiograph 800 through the first electrocardiogram input connector 77 or the cardiac potential information (electrocardiogram) sent directly from the cardiac potential measurement means 900 through the second electrocardiogram input connector 78, an event (waveform) estimated to be an R wave of the electrocardiogram.

The sensing of an event estimated to be an R wave is performed by, for example, detecting a maximum peak waveform (event) in the cycle immediately preceding a cycle (beat) to be sensed and a maximum peak waveform (event) in the further preceding cycle, calculating an average wave height of these maximum peak waveforms, and detecting that the potential difference has reached a height that is 80% of the average height.

The arithmetic processing unit 75 sequentially senses an event estimated to be an R wave from an electrocardiogram input from the electrocardiograph 800 through the first electrocardiogram input connector 77 or an electrocardiogram input from the cardiac potential measurement means 900 through the second electrocardiogram input connector 78, calculates and updates a heart rate each time sensing is performed, and, when, after the n-th event (Vₙ) estimated to be an R wave is sensed and after the application execution switch 745 is input, the (n+m)-th event (Vₙ₊ₘ) estimated to be an R wave is sensed, performs arithmetic processing so that voltages are applied to the first electrode group and the second electrode group in synchronization with the event (Vₙ₊ₘ) only in a case where the event (Vₙ₊ₘ) is sensed after a lapse of a refractory period that begins at the sensing time point of the event (Vₙ) and whose length corresponds to 50% of the reciprocal of a heart rate (Aₙ) at the sensing time point of the event (Vₙ), that is, in a case where the period of time from when the event (Vₙ) is sensed to when the event (Vₙ₊ₘ) is sensed exceeds the length of the refractory period, to control the DC power supply unit.

The refractory period caused by the event (Vₙ) begins at the sensing time point of the event (Vₙ), and the length of the refractory period is a length corresponding to 50% of the reciprocal of the heart rate (Aₙ) at the sensing time point of the event (Vₙ).

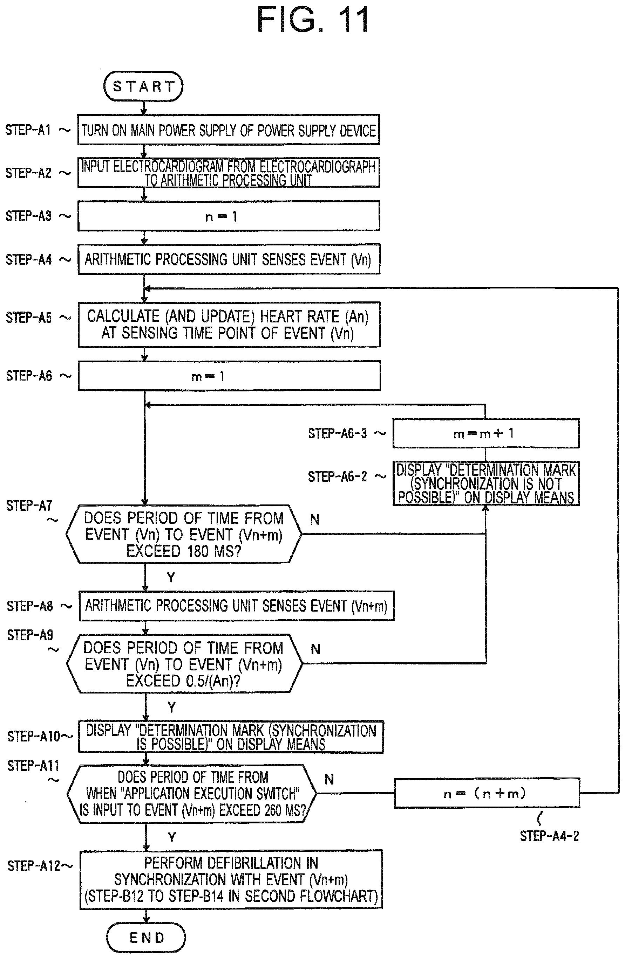

Fig. 11 is a flowchart (hereinafter also referred to as a "first flowchart") illustrating an example of a process for determining, for each of sequentially input events estimated to be R waves by the arithmetic processing unit 75, whether defibrillation can be performed in synchronization with the event.

(1) A main power supply (not illustrated) of the power supply device 700 is turned on [STEP-A1].
(2) Accordingly, an electrocardiogram output from the electrocardiograph 800 or the cardiac potential measurement means 900 is input to the arithmetic processing unit 75 [STEP-A2].
(3) For an event (Vₙ) estimated to be an R wave in the electrocardiogram input to the arithmetic processing unit 75, "1" is first generated as a number (n) indicating the number of times an event has been input since the main power supply was turned on [STEP-A3].
   Note that if "NO" is determined in STEP-A11 below, the number (n) is updated to a number (n+m) indicating an event (Vₙ₊ₘ) in STEP-A11 [STEP-A4-2].
(4) The arithmetic processing unit 75 senses an event (Vₙ) estimated to be an R wave from the input electrocardiogram [STEP-A4].
   Note that if the number (n) is not 1, to sense the event (Vₙ), it is required that a period of 180 ms have elapsed since the immediately preceding event was sensed.
(5) The arithmetic processing unit 75 calculates the heart rate (Aₙ) at the time point when the event (Vₙ) is sensed, and updates it to the calculated heart rate (Aₙ) if the number (n) is not 1 [STEP-A5].

The heart rate (Aₙ) at the sensing time point of the event (Vₙ) is calculated by sampling 12 consecutive events including the event (Vₙ) [events (Vₙ₋₁₁) to (Vₙ)], and is calculated from the period of time from the event (Vₙ₋₁₁) to the event (Vₙ).

If the number of input events including the event (Vₙ) is less than 12, all the events are sampled to calculate the heart rate (Aₙ).

Note that the method for calculating the heart rate (Aₙ) is not limited to this.

(6) For an event (Vₙ₊ₘ) estimated to be an R wave in the electrocardiogram input to the arithmetic processing unit 75, "1" is first generated as a number (m) indicating the number of times an event has been input since the event (Vₙ) was sensed [STEP-A6].

(7) The arithmetic processing unit 75 determines whether the period of time from when the event (Vₙ) is sensed to when the next event (Vₙ₊ₘ) estimated to be an R wave appears exceeds 180 ms; if the period exceeds 180 ms, the process proceeds to STEP-A8; if the period does not exceed 180 ms, then, in STEP-A6-2, the arithmetic processing unit 75 displays, together with a cardiac potential waveform indicating the event (Vₙ₊ₘ) displayed on the display means 79, a determination mark indicating that it is "not possible" to apply a voltage in synchronization with this cardiac potential waveform, and, in STEP-A6-3, sets a number (m+1) obtained by adding 1 to m as new m; and then the process returns to STEP-A7 [STEP-A7].

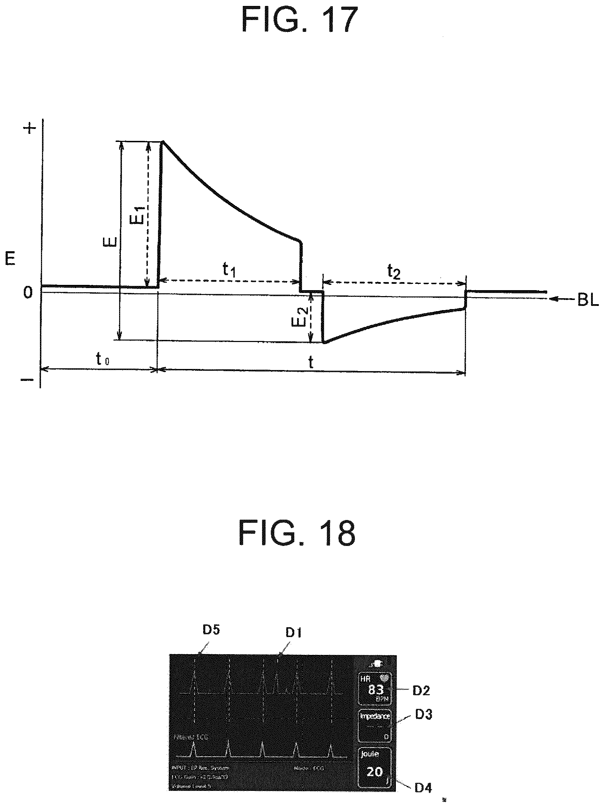

In Fig. 18 illustrating an example of a screen of the display means 79, D1 is a determination mark indicating that it is "not possible" to apply a voltage in synchronization with the cardiac potential waveform presented therebelow.

Furthermore, D2 is a display portion of a heart rate, D3 is a display portion of a value of impedance, and D4 is a display portion of a value of set energy.

(8) The arithmetic processing unit 75 senses the input event (Vₙ₊ₘ) [STEP-A8] .

(9) The arithmetic processing unit 75 determines whether the period of time from when the event (Vₙ) is sensed to when the event (Vₙ₊ₘ) is sensed exceeds 50% (refractory period) of the reciprocal of the heart rate (Aₙ) at the sensing time point of the event (Vₙ); if the period exceeds it, the process proceeds to STEP-A10; if the period does not exceed it, then, in STEP-A6-2, the arithmetic processing unit 75 displays, together with a cardiac potential waveform indicating the event (Vₙ₊ₘ) displayed on the display means 79, a determination mark indicating that it is "not possible" to apply a voltage in synchronization with this cardiac potential waveform, and, in STEP-A6-3, sets a number (m+1) obtained by adding 1 to m as new m; and then the process returns to STEP-A7 [STEP-A9].

(10) The arithmetic processing unit 75 recognizes that it is possible to apply a voltage in synchronization with the sensed event (Vₙ₊ₘ) (the event (Vₙ₊ₘ) is a trigger point), and displays, together with a cardiac potential waveform indicating the event (Vₙ₊ₘ) displayed on the display means 79, a determination mark (in Fig. 18, a vertical-dotted-line determination mark as indicated by D5) indicating that it is possible to apply a voltage in synchronization with this cardiac potential waveform [STEP-A10].

(11) The arithmetic processing unit 75 determines whether the period of time from when the application execution switch 745 is input to when the event (Vₙ₊ₘ) is sensed exceeds 260 ms; if the period exceeds it, the process proceeds to STEP-A12; if the period does not exceed it, then, in STEP-A4-2, the arithmetic processing unit 75 sets (n+m) as new (n); and then the process returns to STEP-A5 [STEP-A11].

(12) Defibrillation is performed in synchronization with the event (Vₙ₊ₘ) [STEP-A12].

Specifically, STEP-B12 to STEP-B14 in a flowchart (second flowchart) described below are executed.

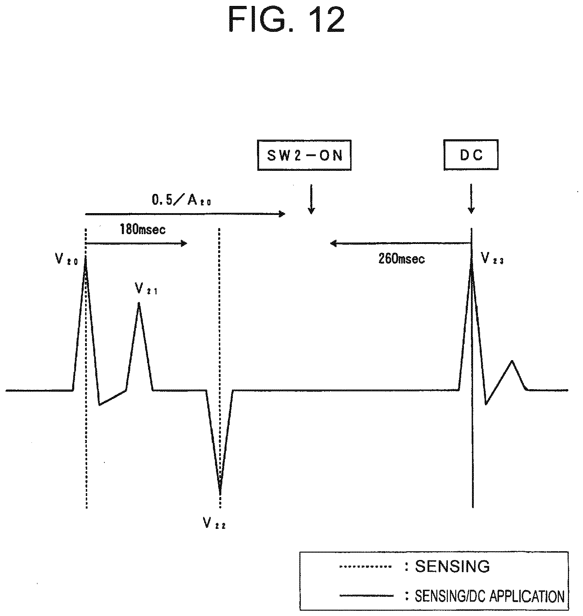

Fig. 12 illustrates the timing of inputting the energy application execution switch 745 and the timing of applying a direct-current voltage in an electrocardiogram input to the arithmetic processing unit 75.

In Fig. 12, an arrow [SW2-ON] represents the time point when the energy application execution switch 745 is input, and an arrow [DC] represents the time point when a direct-current voltage is applied.

Note that, although not illustrated in the drawing, the energy application preparation switch 744 is input before the energy application execution switch 745 is input.

In the electrocardiogram illustrated in Fig. 12, four events (V₂₀) to (V₂₃) estimated to be R waves are recognized.

As illustrated in Fig. 12, the event (V₂₁) is not sensed because it appears within 180 ms after the event (V₂₀) is sensed. However, this event (V₂₁) can be sampled to calculate the heart rate at the sensing time point of the subsequent event.

The event (V₂₂) is sensed because it appears after a lapse of 180 ms after the event (V₂₀) is sensed. However, the event (V₂₂) appears (is sensed) during a refractory period [0.5/ (A₂₀)] caused by the sensing of the event (V₂₀) and thus is not recognized as a trigger point. Therefore, no direct-current voltage is applied in synchronization with this event (V₂₂).

The event (V₂₃) appears (is sensed) after a lapse of the refractory period [0.5/(A₂₀)] caused by the sensing of the event (V₂₀) and is thus recognized as a trigger point. Further, since the period of time from when the energy application execution switch 745 is input to when the event (V₂₃) is sensed exceeds 260 ms, a direct-current voltage is applied in synchronization with the event (V₂₃).

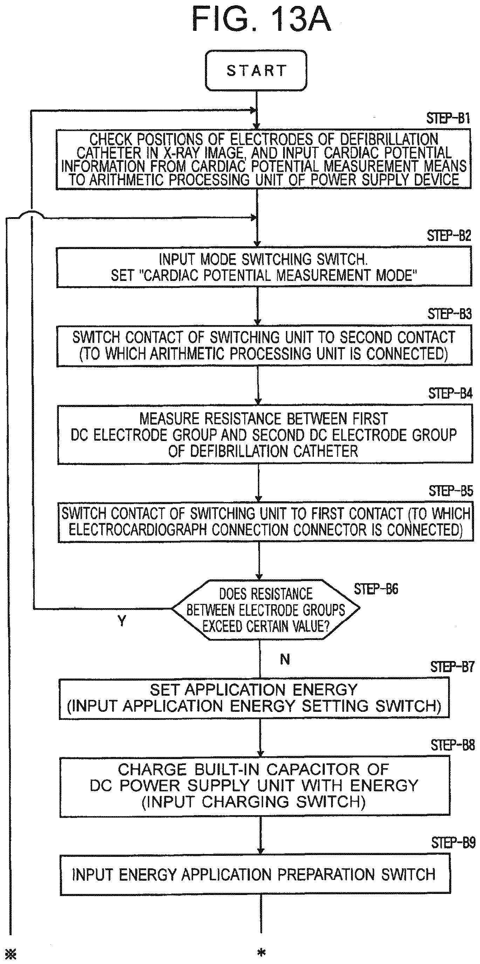

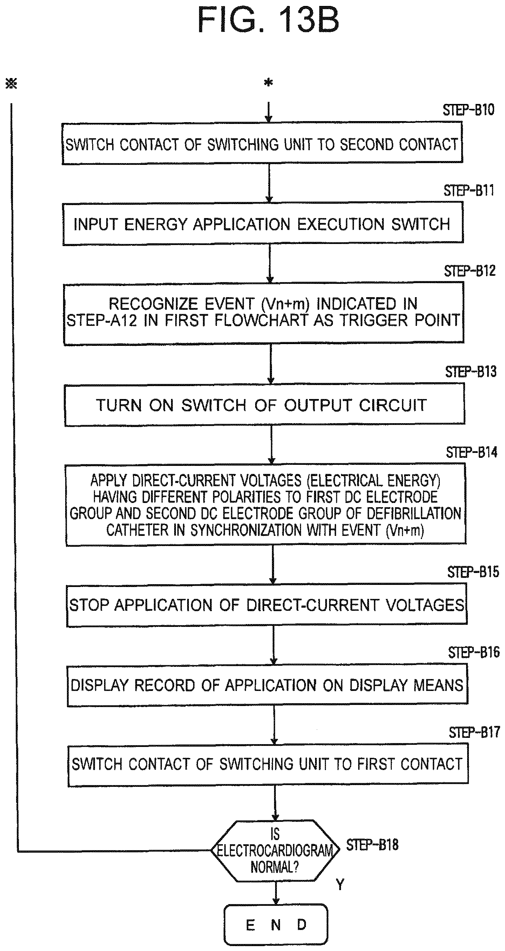

Fig. 13 (Fig. 13A and Fig. 13B) is a flowchart (hereinafter also referred to as a "second flowchart") illustrating an example of defibrillation treatment in the intracardiac defibrillation catheter system of this embodiment.

Note that in this example, an electrocardiogram is input from the electrocardiograph 800 to the arithmetic processing unit 75 through the first electrocardiogram input connector 77.

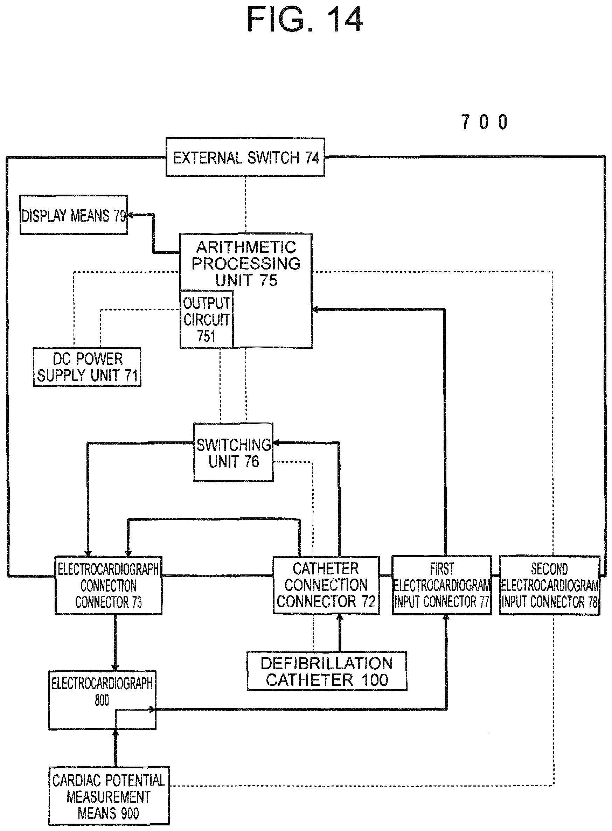

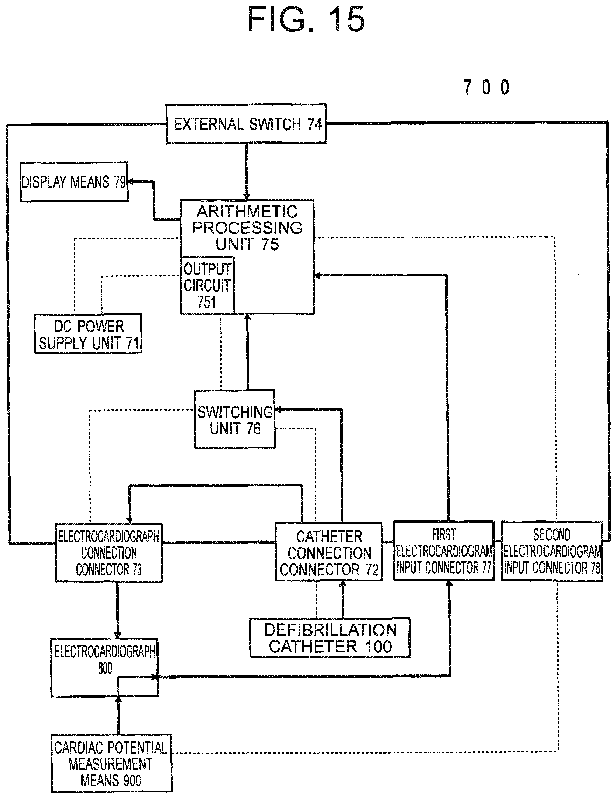

(1) First, the positions of the electrodes of the defibrillation catheter 100 (the electrodes constituting the first DC electrode group 31G, the second DC electrode group 32G, and the proximal-end-side potential measurement electrode group 33G) in an X-ray image are checked, and a portion of cardiac potential information (12-lead electrocardiogram) input from the cardiac potential measurement means 900 (electrode pad attached to the surface of the body) to the electrocardiograph 800 is selected and input from the first electrocardiogram input connector 77 to the arithmetic processing unit 75 of the power supply device 700 [STEP-B1].
   At this time, the portion of the cardiac potential information input to the arithmetic processing unit 75 is displayed on the display means 79 (see Fig. 14). Furthermore, cardiac potential information input from the electrodes constituting the first DC electrode group 31G and/or the second DC electrode group 32G of the defibrillation catheter 100 to the electrocardiograph 800 through the catheter connection connector 72, the switching unit 76, and the electrocardiograph connection connector 73, and cardiac potential information input from the electrodes constituting the proximal-end-side potential measurement electrode group 33G of the defibrillation catheter 100 to the electrocardiograph 800 through the catheter connection connector 72 and the electrocardiograph connection connector 73 are displayed on the monitor (not illustrated) of the electrocardiograph 800.
(2) Then, the mode switching switch 741, which is the external switch 74, is input. The power supply device 700 in this embodiment is set to the "cardiac potential measurement mode" in an initial state, and the switching unit 76 selects the first contact such that a path from the catheter connection connector 72 to the electrocardiograph connection connector 73 through the switching unit 76 is ensured. The mode is set to the "defibrillation mode" in response to an input of the mode switching switch 741 [STEP-B2].
(3) As illustrated in Fig. 15, when the mode switching switch 741 is input and the mode is switched to the defibrillation mode, the contact of the switching unit 76 is switched to the second contact according to a control signal from the arithmetic processing unit 75 such that a path from the catheter connection connector 72 to the arithmetic processing unit 75 through the switching unit 76 is ensured, whereas the path from the catheter connection connector 72 to the electrocardiograph connection connector 73 through the switching unit 76 is blocked [STEP-B3].
   While the switching unit 76 selects the second contact, the cardiac potential information from the electrodes constituting the first DC electrode group 31G and the second DC electrode group 32G of the defibrillation catheter 100 cannot be input to the electrocardiograph 800 (therefore, this cardiac potential information cannot also be sent to the arithmetic processing unit 75). However, the cardiac potential information from the electrodes constituting the proximal-end-side potential measurement electrode group 33G without passing through the switching unit 76 is input to the electrocardiograph 800.
(4) At the time when the contact of the switching unit 76 is switched to the second contact, the resistance between the first DC electrode group (31G) and the second DC electrode group (32G) of the defibrillation catheter 100 is measured [STEP-B4].
   The resistance value input from the catheter connection connector 72 to the arithmetic processing unit 75 through the switching unit 76 is displayed on the display means 79 together with the portion of the cardiac potential information from the cardiac potential measurement means 900, which is input to the arithmetic processing unit 75 (see Fig. 15).
(5) The contact of the switching unit 76 is switched to the first contact, and the path from the catheter connection connector 72 to the electrocardiograph connection connector 73 through the switching unit 76 is recovered [STEP-B5].
   Note that the period of time during which the second contact is selected as the contact of the switching unit 76 (from STEP-B3 to STEP-B5 above) is set to, for example, 1 second.
(6) The arithmetic processing unit 75 determines whether the resistance measured in STEP-B4 exceeds a certain value; if the resistance does not exceed the certain value, the process proceeds to the next, STEP-B7 (preparation for applying a direct-current voltage); and if the resistance exceeds the certain value, the process returns to STEP-B1 (checking of the positions of the electrodes of the defibrillation catheter 100) [STEP-B6].

Here, a case where the resistance exceeds the certain value means that the first DC electrode group and/or the second DC electrode group does not reliably come into abutment against a predetermined area (for example, the vessel wall of the coronary sinus or the inner wall of the right atrium). Thus, the process returns to STEP-B1, and the positions of the electrodes need to be readjusted.

In this manner, since a voltage can be applied only when the first DC electrode group and the second DC electrode group of the defibrillation catheter 100 reliably come into abutment against a predetermined area (for example, the vessel wall of the coronary sinus or the inner wall of the right atrium), effective defibrillation treatment can be performed.

(7) The application energy setting switch 742, which is the external switch 74, is input to set application energy for defibrillation [STEP-B7].

According to the electrode device 700 in this embodiment, the application energy can be set in increments of 1 J from 1 J to 30 J.

(8) The charging switch 743, which is the external switch 74, is input to charge the built-in capacitor of the DC power supply unit 71 with energy [STEP-B8].

(9) After the completion of charging, the operator inputs the energy application preparation switch 744, which is the external switch 74 [STEP-B9].

(10) With the arithmetic processing unit 75, the contact of the switching unit 76 is switched to the second contact, and a path from the catheter connection connector 72 to the arithmetic processing unit 75 through the switching unit 76 is ensured, whereas the path from the catheter connection connector 72 to the electrocardiograph connection connector 73 through the switching unit 76 is blocked [STEP-B10].

(11) The operator inputs the energy application execution switch 745, which is the external switch 74 [STEP-B11].

(12) The arithmetic processing unit 75 recognizes the event (Vₙ₊ₘ) in STEP-A12 in the first flowchart as a trigger point, and then the process proceeds to STEP-B13 [STEP-B12].

(13) The switch of the output circuit 751 of the arithmetic processing unit 75 is turned on, and then the process proceeds to STEP-B14 [STEP-B13].

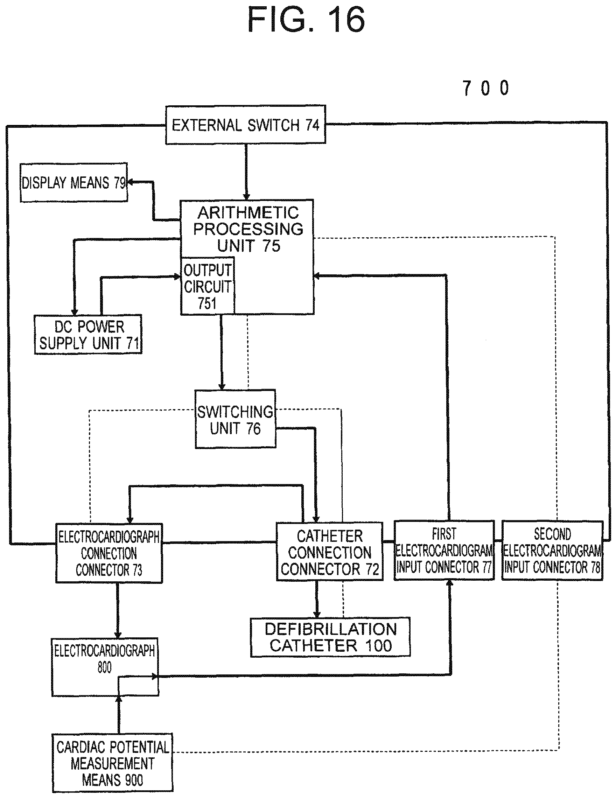

(14) Direct-current voltages having different polarities are applied to the first DC electrode group and the second DC electrode group of the defibrillation catheter 100 from the DC power supply unit 71, which has received a control signal from the arithmetic processing unit 75, through the output circuit 751 of the arithmetic processing unit 75, the switching unit 76, and the catheter connection connector 72 [STEP-B14, see Fig. 16].

Here, the arithmetic processing unit 75 performs arithmetic processing so that direct-current voltages are applied to the first DC electrode group and the second electrode group in synchronization with the event (Vₙ₊ₘ) described above, which is recognized as a trigger point, and sends a control signal to the DC power supply unit 71.

Specifically, the application is started after a lapse of a certain period of time (for example, a very short period of time such as about 1/10 of the peak width of an R wave, which is the event (Vₙ₊ₘ)) after the time point when the event (Vₙ₊ₘ) is sensed (when the next R wave rises).

Fig. 17 is a diagram illustrating a potential waveform measured when the defibrillation catheter 100 in this embodiment applies predetermined electrical energy (for example, set output = 10 J). In this drawing, the horizontal axis represents time, and the vertical axis represents potential.

First, after a certain period of time (t₀) has elapsed since the arithmetic processing unit 75 sensed the event (Vₙ₊ₘ), direct-current voltages are applied to both the first DC electrode group 31G and the second DC electrode group 32G so that the first DC electrode group 31G becomes a minus pole and the second DC electrode group 32G becomes a plus pole, whereby electrical energy is supplied and a measurement potential rises (E₁ is a peak voltage at this time). After a lapse of a certain period of time (t₁), direct-current voltages whose polarities are inverted are applied to both the first DC electrode group 31G and the second DC electrode group 32G so that the first DC electrode group 31G becomes a plus pole and the second DC electrode group 32G becomes a minus pole, whereby electrical energy is supplied and a measurement potential rises (E₂ is a peak voltage at this time).

Here, the period of time (t₀) from when the event (Vₙ₊ₘ) is sensed to when the application is started is set to be, for example, 0.01 to 0.05 seconds, and 0.01 seconds as a preferred example. The period of time (t = t₁ + t₂) is set to be, for example, 0.006 to 0.03 seconds, and 0.02 seconds as a preferred example. Accordingly, a voltage can be applied in synchronization with the event (Vₙ₊ₘ), which is an R wave, and effective defibrillation treatment can be performed.

The peak voltage (E₁) to be measured is set to, for example, 300 to 600 V.

(15) After a certain period of time (t₀+t) has elapsed since the event (Vₙ₊ₘ) was sensed, the application of the voltages from the DC power supply unit 71 is stopped in response to a control signal from the arithmetic processing unit 75 [STEP-B15] .

(16) After the application of the voltages is stopped, a record of the application (such a cardiac potential waveform as illustrated in Fig. 17 during the application) is displayed on the display means 79 [STEP-B16]. The display period of time is set to, for example, 5 seconds.

(17) The contact of the switching unit 76 is switched to the first contact, the path from the catheter connection connector 72 to the electrocardiograph connection connector 73 through the switching unit 76 is recovered, and the cardiac potential information from the electrodes constituting the first DC electrode group 31G and the second DC electrode group 32G of the defibrillation catheter 100 is input to the electrocardiograph 800 [STEP-B17].

(18) The cardiac potential information (electrocardiogram) from the electrodes constituting the defibrillation catheter 100 (the electrodes constituting the first DC electrode group 31G, the second DC electrode group 32G, and the proximal-end-side potential measurement electrode group 33G) and the cardiac potential information from the cardiac potential measurement means 900 (12-lead electrocardiogram), which are displayed on the monitor of the electrocardiograph 800, are observed; if "normal" is indicated, the process ends; and if "not normal (the atrial fibrillation is not cured)" is indicated, the process returns to STEP-B2 [STEP-B18].

According to the intracardiac defibrillation catheter system of this embodiment, after the event (Vₙ) estimated to be an R wave is sensed, even if the event (Vₙ₊ₘ) estimated to be an R wave is sensed during a refractory period caused by this event (Vₙ), no voltage is applied to the first DC electrode group 31G or the second DC electrode group 32G in synchronization with this event (Vₙ₊ₘ). Thus, if the sensed event (Vₙ) is a peak of an R wave, it is possible to avoid performing defibrillation at the time point when the subsequent T wave appears, or, in other words, it is possible to mask a peak estimated to be a T wave.

Additionally, the length of the refractory period caused by the event (Vₙ) is a length [0.5/(Aₙ)] corresponding to 50% of the reciprocal of the heart rate (Aₙ) at the sensing time point of this event (Vₙ), and the refractory period is long when the heart rate (Aₙ) is low, whereas the refractory period is short when the heart rate (Aₙ) is high.

Accordingly, even when the heart rate (Aₙ) is low (the R-R time period is long), no T wave appears after a lapse of the refractory period caused by the event (Vₙ), and a T wave can be reliably masked. It is thus possible to reliably avoid performing defibrillation in synchronization with a T wave.

Furthermore, even when the heart rate (Aₙ) is high (the R-R time period is short), during the refractory period caused by the event (Vₙ), the next event in synchronization with which defibrillation is to be performed does not appear. Thus, defibrillation can be reliably performed in synchronization with an event (R wave) for which defibrillation is to be performed.

According to the intracardiac defibrillation catheter system of this embodiment, therefore, it is possible to reliably avoid performing defibrillation in synchronization with a T wave without being affected by the level of the heart rate of a patient, and it is possible to apply a direct-current voltages to electrodes of the defibrillation catheter 100 to perform defibrillation in synchronization with an R wave of an electrocardiogram input to an arithmetic processing unit.

Furthermore, since the refractory period changes (increases or decreases) on the basis of the heart rate (Aₙ) at the sensing time point of the event (Vₙ), even a rapid change in the heart rate of the patient during surgery can be coped with.

### Reference Signs List

- 100: defibrillation catheter
- 10: multi-lumen tube
- 11: first lumen
- 12: second lumen
- 13: third lumen
- 14: fourth lumen
- 15: fluororesin layer
- 16: inner (core) portion
- 17: outer (shell) portion
- 18: individual stainless steel wire
- 20: handle
- 21: handle body
- 22: knob
- 24: strain relief
- 26: first insulating tube
- 27: second insulating tube
- 28: third insulating tube
- 31G: first DC electrode group
- 31: ring-shaped electrode
- 32G: second DC electrode group
- 32: ring-shaped electrode
- 33G: proximal-end-side potential measurement electrode group
- 33: ring-shaped electrode
- 35: distal end tip
- 41G: first lead wire group
- 41: lead wire
- 42G: second lead wire group
- 42: lead wire
- 43G: third lead wire group
- 43: lead wire
- 50: connector of defibrillation catheter
- 51, 52, 53: pin terminal
- 55: partition plate
- 58: resin
- 61: first protection tube
- 62: second protection tube
- 65: pull wire
- 700: power supply device
- 71: DC power supply unit
- 72: catheter connection connector
- 721, 722, 723: terminal
- 73: electrocardiograph connection connector
- 74: external switch (input means)
- 741: mode switching switch
- 742: application energy setting switch
- 743: charging switch
- 744: energy application preparation switch

- 745: energy application execution switch (discharging switch)
- 75: arithmetic processing unit
- 751: output circuit
- 76: switching unit
- 77: first electrocardiogram input connector
- 78: second electrocardiogram input connector
- 79: display means
- 800: electrocardiograph
- 900: cardiac potential measurement means

## Claims

1. An intracardiac defibrillation catheter system comprising a defibrillation catheter (100) configured to be inserted into a cardiac cavity to perform defibrillation, a power supply device (700) configured to apply a direct-current voltage to electrodes (31, 32, 33) of the defibrillation catheter (100), and an electrocardiograph (800),
wherein the defibrillation catheter (100) comprises an insulating tube member (10),
a first electrode group (31G) composed of a plurality of ring-shaped electrodes (31) mounted in a distal end region of the tube member (10),
a second electrode group (32G) composed of a plurality of ring-shaped electrodes (32) mounted on the tube member (10) at positions spaced away toward a proximal end side from the first electrode group (31G),
a first lead wire group (41G) composed of a plurality of lead wires (41) whose distal ends are connected to the respective electrodes (31) constituting the first electrode group (31G), and
a second lead wire group (42G) composed of a plurality of lead wires (42) whose distal ends are connected to the respective electrodes (32) constituting the second electrode group (32G);
wherein the power supply device (700) comprises a DC power supply unit (71),
a catheter connection connector (72) configured to be connected to a proximal end side of the first lead wire group (41G) and the second lead wire group (42G) of the defibrillation catheter (100), an external switch (74) including an application execution switch (745) for electrical energy, an arithmetic processing unit (75) configured to control the DC power supply unit (71) on the basis of an input of the external switch (74), and
an electrocardiogram input connector (77) configured to be connected to the arithmetic processing unit (75) and an output terminal of the electrocardiograph;
wherein defibrillation is performed with the defibrillation catheter (100) in response to the application execution switch (745) being input, and when defibrillation is to be performed, voltages having different polarities are applied to the first electrode group (31G) and the second electrode group (32G) of the defibrillation catheter (100) from the DC power supply unit (71) through the catheter connection connector (72); and
**characterized in that** the arithmetic processing unit (75) of the power supply device (700) is configured to sequentially sense an event estimated to be an R wave from an electrocardiogram input from the electrocardiograph (800) through the electrocardiogram input connector (77),
calculate and update a heart rate each time sensing is performed, and,
when,
after an n-th event (V n), except for an event sensed during the refractory period caused by a preceding event, estimated to be an R wave is sensed, wherein n is an integer greater than or equal to 1, and
after the application execution switch (745) is input,
an (n+m)-th event (V n+m) estimated to be an R wave is sensed, wherein m is an integer greater than or equal to 1,
perform arithmetic processing so that voltages are applied to the first electrode group (31G) and the second electrode group (32G) in synchronization with the event (V n+m) only in a case where the event (V n+m) is sensed after a lapse of a refractory period that begins at a sensing time point of the event (V n) and whose length changes in proportion to a reciprocal of a heart rate (A n) at the sensing time point of the event (V n), to control the DC power supply unit (71).

2. The intracardiac defibrillation catheter system according to Claim 1, wherein the arithmetic processing unit (75) of the power supply device (700) is configured to calculate the heart rate (A n) at the sensing time point of the event (V n) on the basis of a plurality of consecutive events including the event (V n).

3. The intracardiac defibrillation catheter system according to Claim 1 or 2, wherein the length of the refractory period caused by the event (V n) is a length corresponding to at least 30% and at most 80% of the reciprocal of the heart rate (A n) at the sensing time point of the event (V n).

4. The intracardiac defibrillation catheter system according to Claim 1 or 2, wherein the length of the refractory period caused by the event (V n) is a length corresponding to 50% of the reciprocal of the heart rate (A n) at the sensing time point of the event (V n).

5. The intracardiac defibrillation catheter system according to any of Claims 1 to 4, wherein the arithmetic processing unit (75) of the power supply device is configured to not newly sense an event estimated to be an R wave for a period of at least 10 ms and at most 500 ms after an event estimated to be an R wave is sensed.

6. The intracardiac defibrillation catheter system according to any of Claims 1 to 5, wherein the arithmetic processing unit (75) of the power supply device (700) is configured to control the DC power supply unit (71) so that no voltage is applied to the first electrode group (31G) or the second electrode group (32G) for a period of at least 10 ms and at most 500 ms after an input of the application execution switch (745).

7. The intracardiac defibrillation catheter system according to any of Claims 1 to 6, comprising cardiac potential measurement means (900),
wherein the power supply device (700) is configured to comprise a second electrocardiogram input connector (78) to be connected to the arithmetic processing unit (75) and the cardiac potential measurement means (900); and
wherein when an electrocardiogram is input from the cardiac potential measurement means (900) through the second electrocardiogram input connector (78), the arithmetic processing unit (75) of the power supply device (700)
is configured to sequentially sense an event estimated to be an R wave from the input electrocardiogram, calculate and update a heart rate each time sensing is performed, and,
when, after an n-th event (V n) estimated to be an R wave, except for an event sensed during the refractory period caused by a preceding event, is sensed and
after the application execution switch is input,
an (n+m)-th event (V n+m) estimated to be an R wave is sensed,
perform arithmetic processing so that voltages are applied to the first electrode group (31G) and the second electrode group (32G) in synchronization with the event (V n+m) only in a case where the event (V n+m) is sensed after a lapse of the refractory period that begins at a sensing time point of the event (V n) and whose length changes in proportion to a reciprocal of a heart rate (A n) at the sensing time point of the event (V n), to control the DC power supply unit (71).

8. The intracardiac defibrillation catheter system according to any of Claims 1 to 7, wherein the power supply device (700) has display means (79) for displaying an electrocardiogram input to the arithmetic processing unit (75), and
wherein the display means (75) is configured to display, together with a cardiac potential waveform indicating the event (V n+m) sensed after a lapse of the refractory period caused by the event (V n), a mark indicating that it is possible to apply a voltage in synchronization with this cardiac potential waveform, regardless of whether the application execution switch (745) is input.

9. The intracardiac defibrillation catheter system according to Claim 8, wherein the display means (75) is configured to display, together with a cardiac potential waveform indicating the event sensed during the refractory period caused by the event (V n), a mark indicating that it is not possible to apply a voltage in synchronization with this cardiac potential waveform.

## Patentansprüche

1. Ein intrakardiales Defibrillationskathetersystem, umfassend einen Defibrillationskatheter (100), der konfiguriert ist, um in eine Herzhöhle eingeführt zu werden, um eine Defibrillation durchzuführen, eine Leistungsversorgungsvorrichtung (700), die konfiguriert ist, um eine Gleichspannung an Elektroden (31, 32, 33) des Defibrillationskatheters (100) anzulegen, und einen Elektrokardiografen (800),
wobei der Defibrillationskatheter (100) umfasst:
ein Isolierrohrelement (10),
eine erste Elektrodengruppe (31G), die aus einer Vielzahl von ringförmigen Elektroden (31) besteht, die in einer distalen Endregion des Rohrelements (10) montiert sind,
eine zweite Elektrodengruppe (32G), die aus einer Vielzahl von ringförmigen Elektroden (32) besteht, die an dem Rohrelement (10) an Positionen montiert sind, die zu einer proximalen Endseite von der ersten Elektrodengruppe (31G) beabstandet sind,
eine erste Zuleitungsdrahtgruppe (41G), die aus einer Vielzahl von Zuleitungsdrähten (41) besteht, deren distale Enden mit den jeweiligen Elektroden (31) verbunden sind, die die erste Elektrodengruppe (31G) bilden, und
eine zweite Zuleitungsdrahtgruppe (42G), die aus einer Vielzahl von Zuleitungsdrähten (42) besteht, deren distale Enden mit den jeweiligen Elektroden (32) verbunden sind, die die zweite Elektrodengruppe (32G) bilden;
wobei die Leistungsversorgungsvorrichtung (700) umfasst:
eine Gleichstromversorgungseinheit (71),
einen Katheterverbindungskonnektor (72), der konfiguriert ist, um mit einer proximalen Endseite der ersten Zuleitungsdrahtgruppe (41G) und der zweiten Zuleitungsdrahtgruppe (42G) des Defibrillationskatheters (100) verbunden zu werden,
einen externen Schalter (74), einschließlich eines Anlegungsausführungsschalters (745) für elektrische Energie,
eine arithmetische Verarbeitungseinheit (75), die konfiguriert ist, um die Gleichstromversorgungseinheit (71) auf der Basis einer Eingabe des externen Schalters (74) zu steuern, und
einen Elektrokardiogrammeingangskonnektor (77), der konfiguriert ist, um mit der arithmetischen Verarbeitungseinheit (75) und einem Ausgangsanschluss des Elektrokardiografen verbunden zu werden;
wobei die Defibrillation mit dem Defibrillationskatheter (100) durchgeführt wird, als Reaktion auf den Anlegungsausführungsschalter (745), die eingegeben wird, und wenn die Defibrillation durchgeführt werden soll, Spannungen, die unterschiedliche Polaritäten aufweisen, auf die erste Elektrodengruppe (31G) und die zweite Elektrodengruppe (32G) des Defibrillationskatheters (100) aus der Gleichstromversorgungseinheit (71) durch den Katheterverbindungskonnektor (72) angelegt werden; und
**dadurch gekennzeichnet, dass** die arithmetische Verarbeitungseinheit (75) der Leistungsversorgungsvorrichtung (700) konfiguriert ist, um ein Ereignis, das als eine R-Welle eingeschätzt wird, aus einem Elektrokardiogrammeingang aus dem Elektrokardiografen (800) durch den Elektrokardiogrammeingangskonnektor (77) sequenziell zu erfassen,
eine Herzfrequenz jedes Mal, wenn eine Erfassung durchgeführt wird, zu berechnen und zu aktualisieren, und,
wenn,
nach einem n-ten Ereignis (V n), außer für ein Ereignis, das während des Refraktärzeitraums erfasst wird, das durch ein vorhergehendes Ereignis verursacht wird, das als eine R-Welle eingeschätzt wird, erfasst wird, wobei n eine ganze Zahl größer oder gleich 1 ist, und
nachdem der Anlegungsausführungsschalter (745) eingegeben wurde,
ein (n+m)-tes Ereignis (V n+m), das als eine R-Welle eingeschätzt wird, erfasst wird, wobei m eine ganze Zahl größer oder gleich 1 ist,
Durchführen einer arithmetischen Verarbeitung, sodass Spannungen, die an die erste Elektrodengruppe (31G) und die zweite Elektrodengruppe (32G) in Synchronisation mit dem Ereignis (V n+m) nur in einem Fall angelegt werden, in dem das Ereignis (V n+m) nach einem Ablauf eines Refraktärzeitraums erfasst wird, der zu einem Erfassungszeitpunkt des Ereignisses (V n) beginnt, und dessen Länge sich proportional zu einem Kehrwert einer Herzfrequenz (A n) zu dem Erfassungszeitpunkt des Ereignisses (V n) ändert,
um die Gleichstromversorgungseinheit (71) zu steuern.

2. Intrakardiales Defibrillationskathetersystem nach Anspruch 1, wobei die arithmetische Verarbeitungseinheit (75) der Leistungsversorgungsvorrichtung (700) konfiguriert ist, um die Herzfrequenz (A n) zu dem Erfassungszeitpunkt des Ereignisses (V n) auf der Basis einer Vielzahl von aufeinanderfolgenden Ereignissen einschließlich des Ereignisses (V n) zu berechnen.

3. Intrakardiales Defibrillationskathetersystem nach Anspruch 1 oder 2, wobei die Länge des Refraktärzeitraums, der durch das Ereignis (V n) verursacht wird, eine Länge aufweist, die mindestens 30 % und höchstens 80 % des Kehrwerts der Herzfrequenz (A n) zu dem Erfassungszeitpunkt des Ereignisses (V n) entspricht.

4. Intrakardiales Defibrillationskathetersystem nach Anspruch 1 oder 2, wobei die Länge des Refraktärzeitraums, der durch das Ereignis (V n) verursacht wird, eine Länge aufweist, die 50 % des Kehrwerts der Herzfrequenz (A n) zu dem Erfassungszeitpunkt des Ereignisses (V n) entspricht.

5. Intrakardiales Defibrillationskathetersystem nach einem der Ansprüche 1 bis 4, wobei die arithmetische Verarbeitungseinheit (75) der Leistungsversorgungsvorrichtung konfiguriert ist, um ein Ereignis, das als eine R-Welle eingeschätzt wird, für einen Zeitraum von mindestens 10 ms und höchstens 500 ms nicht neu zu erfassen, nachdem ein Ereignis erfasst wurde, das als eine R-Welle eingeschätzt wird.

6. Intrakardiales Defibrillationskathetersystem nach einem der Ansprüche 1 bis 5, wobei die arithmetische Verarbeitungseinheit (75) der Leistungsversorgungsvorrichtung (700) konfiguriert ist, um die Gleichstromversorgungseinheit (71) zu steuern, sodass keine Spannung an die erste Elektrodengruppe (31G) oder die zweite Elektrodengruppe (32G) für einen Zeitraum von mindestens 10 ms und höchstens 500 ms nach einer Eingabe des Anlegungsausführungsschalters (745) angelegt wird.

7. Intrakardiales Defibrillationskathetersystem nach einem der Ansprüche 1 bis 6, umfassend Herzpotenzialmessmittel (900),
wobei die Leistungsversorgungsvorrichtung (700) konfiguriert ist, um einen zweiten Elektrokardiogrammeingangskonnektor (78) zu umfassen, der mit der arithmetischen Verarbeitungseinheit (75) und dem Herzpotenzialmessmittel (900) zu verbinden ist; und
wobei, wenn ein Elektrokardiogramm von dem Herzpotenzialmessmittel (900) durch den zweiten Elektrokardiogrammeingangskonnektor (78) eingegeben wird, die arithmetische Verarbeitungseinheit (75) der Leistungsversorgungsvorrichtung (700) konfiguriert ist, um ein Ereignis, das als eine R-Welle eingeschätzt wird, aus dem Eingabeelektrokardiogramm sequenziell zu erfassen, eine Herzfrequenz jedes Mal, wenn eine Erfassung durchgeführt wird, zu berechnen und zu aktualisieren, und,
wenn nach einem n-ten Ereignis (V n), das als eine R-Welle eingeschätzt wird, außer für ein Ereignis, das während des Refraktärzeitraums erfasst wird, das durch ein vorhergehendes Ereignis verursacht wird, erfasst wird, und
nachdem der Anlegungsausführungsschalter eingegeben wurde,
ein (n+m)-tes Ereignis (V n+m), das als eine R-Welle eingeschätzt wird, erfasst wird,
Durchführen einer arithmetischen Verarbeitung, sodass Spannungen an die erste Elektrodengruppe (31G) und die zweite Elektrodengruppe (32G) in Synchronisation mit dem Ereignis (V n+m) nur in einem Fall angelegt werden, in dem das Ereignis (V n+m) nach einem Ablauf des Refraktärzeitraums erfasst wird, der zu einem Erfassungszeitpunkt des Ereignisses (V n) beginnt, und dessen Länge sich proportional zu einem Kehrwert einer Herzfrequenz (A n) zu dem Erfassungszeitpunkt des Ereignisses (V n) ändert, um die Gleichstromversorgungseinheit (71) zu steuern.

8. Intrakardiales Defibrillationskathetersystem nach einem der Ansprüche 1 bis 7, wobei die Leistungsversorgungsvorrichtung (700) Anzeigemittel (79) zum Anzeigen eines Elektrokardiogrammeingangs in die arithmetische Verarbeitungseinheit (75) aufweist, und
wobei das Anzeigemittel (75) konfiguriert ist, zusammen mit einer Herzpotenzial-Wellenform, die das Ereignis (V n+m), das nach Ablauf des durch das Ereignis (V n) verursachten Refraktärzeitraums, erfasst wird, anzuzeigen, wobei eine Markierung angibt, dass es möglich ist, Spannung in Synchronisation mit dieser Herzpotential-Wellenform anzulegen, ungeachtet dessen, ob der Anwendungsausführungsschalter (745) eingegeben ist.

9. Intrakardiales Defibrillationskathetersystem nach Anspruch 8, wobei das Anzeigemittel (75) konfiguriert ist, um zusammen mit einer Herzpotential-Wellenform, die das Ereignis, das während dem durch das Ereignis (V n) verursachten Refraktärzeitraums erfasst wird, anzuzeigen, wobei eine Markierung angibt, dass es nicht möglich ist, eine Spannung in Synchronisation mit dieser Herzpotential-Wellenform anzulegen.

## Revendications

1. Système de cathéter de défibrillation intracardiaque comprenant un cathéter de défibrillation (100) configuré pour être inséré dans une cavité cardiaque pour effectuer une défibrillation, un dispositif d'alimentation électrique (700) configuré pour appliquer une tension continue aux électrodes (31, 32, 33) du cathéter de défibrillation (100), et un électrocardiographe (800),
dans lequel le cathéter de défibrillation (100) comprend
un élément de tube isolant (10),
un premier groupe d'électrodes (31G) composé d'une pluralité d'électrodes en forme d'anneau (31) montées dans une région d'extrémité distale de l'élément tubulaire (10),
un second groupe d'électrodes (32G) composé d'une pluralité d'électrodes en forme d'anneau (32) montées sur l'élément tubulaire (10) à des positions espacées vers un côté d'extrémité proximale du premier groupe d'électrodes (31G),
un premier groupe de fils conducteurs (41G) composé d'une pluralité de fils conducteurs (41) dont les extrémités distales sont connectées aux électrodes respectives (31) constituant le premier groupe d'électrodes (31G), et
un second groupe de fils conducteurs (42G) composé d'une pluralité de fils conducteurs (42) dont les extrémités distales sont connectées aux électrodes respectives (32) constituant le second groupe d'électrodes (32G) ;
dans lequel le dispositif d'alimentation électrique (700) comprend
un bloc d'alimentation CC (71),
un connecteur de connexion de cathéter (72) configuré pour être connecté à un côté d'extrémité proximale du premier groupe de fils conducteurs (41G) et du second groupe de fils conducteurs (42G) du cathéter de défibrillation (100),
un commutateur externe (74) comportant un commutateur d'exécution d'application (745) pour l'énergie électrique,
une unité de traitement arithmétique (75) configurée pour commander le bloc d'alimentation CC (71) sur la base d'une entrée du commutateur externe (74), et
un connecteur d'entrée d'électrocardiogramme (77) configuré pour être connecté à l'unité de traitement arithmétique (75) et une borne de sortie de l'électrocardiographe ;
dans lequel la défibrillation est effectuée avec le cathéter de défibrillation (100) en réponse à l'entrée du commutateur d'exécution d'application (745), et lorsque la défibrillation doit être effectuée, des tensions ayant des polarités différentes sont appliquées au premier groupe d'électrodes (31G) et au second groupe d'électrodes (32G) du cathéter de défibrillation (100) à partir du bloc d'alimentation CC (71) à travers le connecteur de connexion de cathéter (72) ; et
**caractérisé en ce que** l'unité de traitement arithmétique (75) du dispositif d'alimentation électrique (700) est configurée pour détecter séquentiellement un événement estimé comme étant une onde R provenant d'une entrée d'électrocardiogramme provenant de l'électrocardiographe (800) à travers le connecteur d'entrée d'électrocardiogramme (77),
calculer et mettre à jour une fréquence cardiaque à chaque fois qu'une détection est effectuée, et,
lorsque,
après la détection d'un n-ème événement (V n), à l'exception d'un événement détecté pendant la période réfractaire provoquée par un événement précédent, estimé comme étant une onde R, dans lequel n est un nombre entier supérieur ou égal à 1, et
après l'entrée du commutateur d'exécution d'application (745),
un (n + m)-ème événement (V n + m) estimé comme étant une onde R est détecté, dans lequel m est un entier supérieur ou égal à 1,
effectuer un traitement arithmétique de sorte que des tensions sont appliquées au premier groupe d'électrodes (31G) et au second groupe d'électrodes (32G) en synchronisation avec l'événement (V n + m) seulement dans un cas où l'événement (V n + m) est détecté après un laps de temps d'une période réfractaire qui commence au niveau d'un point temporel de détection de l'événement (V n) et dont la longueur change proportionnellement à une réciproque d'une fréquence cardiaque (A n) au niveau du point temporel de détection de l'événement (V n),
pour commander le bloc d'alimentation CC (71).

2. Système de cathéter de défibrillation intracardiaque selon la revendication 1, dans lequel l'unité de traitement arithmétique (75) du dispositif d'alimentation électrique (700) est configurée pour calculer la fréquence cardiaque (A n) au niveau du point temporel de détection de l'événement (V n) sur la base d'une pluralité d'événements consécutifs comportant l'événement (V n).

3. Système de cathéter de défibrillation intracardiaque selon la revendication 1 ou 2, dans lequel la longueur de la période réfractaire provoquée par l'événement (V n) est une longueur correspondant à au moins 30 % et au plus 80 % de la réciproque du rythme cardiaque (A n) au niveau du point temporel de détection de l'événement (V n).

4. Système de cathéter de défibrillation intracardiaque selon la revendication 1 ou 2, dans lequel la longueur de la période réfractaire provoquée par l'événement (V n) est une longueur correspondant à 50 % de la réciproque du rythme cardiaque (A n) au niveau du point temporel de détection de l'événement (V n).

5. Système de cathéter de défibrillation intracardiaque selon l'une quelconque des revendications 1 à 4, dans lequel l'unité de traitement arithmétique (75) du dispositif d'alimentation électrique est configurée pour ne pas nouvellement détecter un événement estimé comme étant une onde R pendant une période d'au moins 10 ms et d'au plus 500 ms après la détection d'un événement estimé comme étant une onde R.

6. Système de cathéter de défibrillation intracardiaque selon l'une quelconque des revendications 1 à 5, dans lequel l'unité de traitement arithmétique (75) du dispositif d'alimentation électrique (700) est configurée pour commander le bloc d'alimentation CC (71) de sorte qu'aucune tension n'est appliquée au premier groupe d'électrodes (31G) ou au second groupe d'électrodes (32G) pendant une période d'au moins 10 ms et d'au plus 500 ms après une entrée du commutateur d'exécution d'application (745).

7. Système de cathéter de défibrillation intracardiaque selon l'une quelconque des revendications 1 à 6, comprenant un moyen de mesure de potentiel cardiaque (900),
dans lequel le dispositif d'alimentation électrique (700) est configuré pour comprendre un second connecteur d'entrée d'électrocardiogramme (78) à connecter à l'unité de traitement arithmétique (75) et au moyen de mesure de potentiel cardiaque (900) ; et
dans lequel, lorsqu'un électrocardiogramme est entré par le moyen de mesure de potentiel cardiaque (900) à travers le second connecteur d'entrée d'électrocardiogramme (78), l'unité de traitement arithmétique (75) du dispositif d'alimentation électrique (700) est configurée pour détecter séquentiellement un événement estimé comme étant une onde R provenant de l'électrocardiogramme d'entrée, calculer et mettre à jour une fréquence cardiaque à chaque fois qu'une détection est effectuée, et,
lorsque, après la détection d'un n-ème événement (V n) estimé comme étant une onde R, à l'exception d'un événement détecté pendant la période réfractaire provoquée par un événement précédent, et
après l'entrée du commutateur d'exécution d'application,
un (n + m)-ème événement (V n + m) estimé comme une onde R est détecté,
effectuer un traitement arithmétique de sorte que des tensions sont appliquées au premier groupe d'électrodes (31G) et au second groupe d'électrodes (32G) en synchronisation avec l'événement (V n + m) seulement dans un cas où l'événement (V n + m) est détecté après un laps de temps de la période réfractaire qui commence au niveau d'un point temporel de détection de l'événement (V n) et dont la longueur change proportionnellement à une réciproque d'une fréquence cardiaque (A n) au niveau du point temporel de détection de l'événement (V n), pour commander le bloc d'alimentation CC (71).

8. Système de cathéter de défibrillation intracardiaque selon l'une quelconque des revendications 1 à 7, dans lequel le dispositif d'alimentation électrique (700) a un moyen d'affichage (79) pour afficher une entrée d'électrocardiogramme à l'unité de traitement arithmétique (75), et
dans lequel le moyen d'affichage (75) est configuré pour afficher, conjointement avec une forme d'onde de potentiel cardiaque indiquant l'événement (V n + m) détecté après un laps de temps de la période réfractaire provoquée par l'événement (V n), une marque indiquant qu'il est possible d'appliquer une tension en synchronisation avec cette forme d'onde de potentiel cardiaque, indépendamment de l'entrée du commutateur d'exécution d'application (745).

9. Système de cathéter de défibrillation intracardiaque selon la revendication 8, dans lequel le moyen d'affichage (75) est configuré pour afficher, conjointement avec une forme d'onde de potentiel cardiaque indiquant l'événement détecté pendant la période réfractaire provoquée par l'événement (V n), une marque indiquant qu'il n'est pas possible d'appliquer une tension en synchronisation avec cette forme d'onde de potentiel cardiaque.
